# EUROPEAN PATENT APPLICATION

(11) **EP 3 725 299 A1**
(43) Date of publication of application: **21.10.2020**
(21) Application number: 20156624.7
(22) Date of filing: 21.06.2006
(51) Int. Cl.: A61K 9/00, A61K 47/42, A61K 31/138, A61K 31/165, A61K 31/196, A61K 31/198, A61K 31/277, A61K 31/337, A61K 31/40, A61K 31/4745, A61K 31/475, A61K 31/513, A61K 31/519, A61K 31/675, A61K 31/7028, A61K 31/704, A61K 31/7048, A61K 31/7068, A61K 33/243, A61K 38/08, A61P 35/00

(54) **A DIRECT DRUG DELIVERY SYSTEM BASED ON THERMALLY RESPONSIVE BIOPOLYMERS**

(30) Priority: 24.06.2005 US 69396605 P
(62) Divisional of application: 13176325.2
(71) Applicant: DUKE UNIVERSITY, Durham NC 27707 (US)
(72) Inventor: SETTON, Lori, A, Malvern, Pennsylvania 19355 (US); CHILKOTI, Ashutosh, Durham, North Carolina 27701 (US); KRAUS, Virginia, B, Hillsborough, North Carolina 27278 (US); BETRE, Helawe, Malvern, Pennsylvania 19355 (US); DREHER, Matthew R., Malvern, PA Pennsylvania 19355 (US)
(74) Representative: Cooley (UK) LLP

(57) **Abstract**

A method for delivering a drug depot of a compound of interest to a selected region in a subject. The method comprises administering a composition directly to said region of interest, the composition comprising the compound of interest to be delivered (such as an antiinflammatory agent or a chemotherapeutic agent) and a polymer (such as an elastin-like peptide or ELP) that undergoes an inverse temperature phase transition, so that a sustained release of the compound of interest at the selected region is provided. Compositions useful for carrying out the invention are also described.

SEQUENCE LISTING

<110> Duke University

<120> A DIRECT DRUG DELIVERY SYSTEM BASED ON THERMALLY RESPONSIVE
      BIOPOLYMERS

<130> PHAS-004/00WO

<140> EP 06785402.6
<141> 2006-06-21

<140> PCT/US2006/024427
<151> 2006-06-21

<150> US 60/693,966
<151> 2005-06-24

<160> 25

<170> PatentIn version 3.3

<210> 1
<211> 450
<212> PRT
<213> Artificial

<220>
<223> Thermosensitive bioelastic polymer sequence

<400> 1
Val Pro Gly Val Gly Val Pro Gly Val Gly Val Pro Gly Val Gly Val
1               5                   10                  15
Pro Gly Val Gly Val Pro Gly Val Gly Val Pro Gly Val Gly Val Pro
            20                  25                  30
Gly Val Gly Val Pro Gly Val Gly Val Pro Gly Val Gly Val Pro Gly
        35                  40                  45
Val Gly Val Pro Gly Val Gly Val Pro Gly Val Gly Val Pro Gly Val
    50                  55                  60
Gly Val Pro Gly Val Gly Val Pro Gly Val Gly Val Pro Gly Val Gly
65                  70                  75                  80
Val Pro Gly Val Gly Val Pro Gly Val Gly Val Pro Gly Val Gly Val
                85                  90                  95
Pro Gly Val Gly Val Pro Gly Val Gly Val Pro Gly Val Gly Val Pro
            100                 105                 110
Gly Val Gly Val Pro Gly Val Gly Val Pro Gly Val Gly Val Pro Gly
        115                 120                 125
Val Gly Val Pro Gly Val Gly Val Pro Gly Val Gly Val Pro Gly Val
    130                 135                 140
Gly Val Pro Gly Val Gly Val Pro Gly Val Gly Val Pro Gly Val Gly
145                 150                 155                 160
Val Pro Gly Val Gly Val Pro Gly Val Gly Val Pro Gly Val Gly Val
                165                 170                 175
Pro Gly Val Gly Val Pro Gly Val Gly Val Pro Gly Val Gly Val Pro
            180                 185                 190
Gly Val Gly Val Pro Gly Val Gly Val Pro Gly Val Gly Val Pro Gly
        195                 200                 205
Val Gly Val Pro Gly Val Gly Val Pro Gly Val Gly Val Pro Gly Val
    210                 215                 220
Gly Val Pro Gly Val Gly Val Pro Gly Val Gly Val Pro Gly Val Gly
225                 230                 235                 240
Val Pro Gly Val Gly Val Pro Gly Val Gly Val Pro Gly Val Gly Val
                245                 250                 255
Pro Gly Val Gly Val Pro Gly Val Gly Val Pro Gly Val Gly Val Pro
            260                 265                 270
Gly Val Gly Val Pro Gly Val Gly Val Pro Gly Val Gly Val Pro Gly
        275                 280                 285
Val Gly Val Pro Gly Val Gly Val Pro Gly Val Gly Val Pro Gly Val

290                     295                     300
Gly Val Pro Gly Val Gly Val Pro Gly Val Gly Val Pro Gly Val Gly
305                 310                     315                 320
Val Pro Gly Val Gly Val Pro Gly Val Gly Val Pro Gly Val Gly Val
                325                     330                     335
Pro Gly Val Gly Val Pro Gly Val Gly Val Pro Gly Val Gly Val Pro
            340                     345                     350
Gly Val Gly Val Pro Gly Val Gly Val Pro Gly Val Gly Val Pro Gly
        355                     360                     365
Val Gly Val Pro Gly Val Gly Val Pro Gly Val Gly Val Pro Gly Val
    370                     375                     380
Gly Val Pro Gly Val Gly Val Pro Gly Val Gly Val Pro Gly Val Gly
385                 390                     395                 400
Val Pro Gly Val Gly Val Pro Gly Val Gly Val Pro Gly Val Gly Val
                405                     410                     415
Pro Gly Val Gly Val Pro Gly Val Gly Val Pro Gly Val Gly Val Pro
            420                     425                     430
Gly Val Gly Val Pro Gly Val Gly Val Pro Gly Val Gly Val Pro Gly
        435                     440                     445
Val Gly
    450



<210> 2
<211> 600
<212> PRT
<213> Artificial

<220>
<223> Thermosensitive bioelastic polymer sequence

<400> 2
Val Pro Gly Val Gly Val Pro Gly Val Gly Val Pro Gly Val Gly Val
1               5                   10                  15
Pro Gly Val Gly Val Pro Gly Val Gly Val Pro Gly Val Gly Val Pro
            20                  25                  30
Gly Val Gly Val Pro Gly Val Gly Val Pro Gly Val Gly Val Pro Gly
        35                  40                  45
Val Gly Val Pro Gly Val Gly Val Pro Gly Val Gly Val Pro Gly Val
    50                  55                  60
Gly Val Pro Gly Val Gly Val Pro Gly Val Gly Val Pro Gly Val Gly
65                  70                  75                  80
Val Pro Gly Val Gly Val Pro Gly Val Gly Val Pro Gly Val Gly Val
                85                  90                  95
Pro Gly Val Gly Val Pro Gly Val Gly Val Pro Gly Val Gly Val Pro
            100                 105                 110
Gly Val Gly Val Pro Gly Val Gly Val Pro Gly Val Gly Val Pro Gly
        115                 120                 125
Val Gly Val Pro Gly Val Gly Val Pro Gly Val Gly Val Pro Gly Val
    130                 135                 140
Gly Val Pro Gly Val Gly Val Pro Gly Val Gly Val Pro Gly Val Gly
145                 150                 155                 160
Val Pro Gly Val Gly Val Pro Gly Val Gly Val Pro Gly Val Gly Val
                165                 170                 175
Pro Gly Val Gly Val Pro Gly Val Gly Val Pro Gly Val Gly Val Pro
            180                 185                 190
Gly Val Gly Val Pro Gly Val Gly Val Pro Gly Val Gly Val Pro Gly
        195                 200                 205
Val Gly Val Pro Gly Val Gly Val Pro Gly Val Gly Val Pro Gly Val
    210                 215                 220
Gly Val Pro Gly Val Gly Val Pro Gly Val Gly Val Pro Gly Val Gly
225                 230                 235                 240
Val Pro Gly Val Gly Val Pro Gly Val Gly Val Pro Gly Val Gly Val
                245                 250                 255
Pro Gly Val Gly Val Pro Gly Val Gly Val Pro Gly Val Gly Val Pro
            260                 265                 270
Gly Val Gly Val Pro Gly Val Gly Val Pro Gly Val Gly Val Pro Gly
        275                 280                 285

Val Gly Val Pro Gly Val Gly Val Pro Gly Val Gly Val Pro Gly Val
    290                 295                 300
Gly Val Pro Gly Val Gly Val Pro Gly Val Gly Val Pro Gly Val Gly
305                 310                 315                 320
Val Pro Gly Val Gly Val Pro Gly Val Gly Val Pro Gly Val Gly Val
                325                 330                 335
Pro Gly Val Gly Val Pro Gly Val Gly Val Pro Gly Val Gly Val Pro
            340                 345                 350
Gly Val Gly Val Pro Gly Val Gly Val Pro Gly Val Gly Val Pro Gly
        355                 360                 365
Val Gly Val Pro Gly Val Gly Val Pro Gly Val Gly Val Pro Gly Val
    370                 375                 380
Gly Val Pro Gly Val Gly Val Pro Gly Val Gly Val Pro Gly Val Gly
385                 390                 395                 400
Val Pro Gly Val Gly Val Pro Gly Val Gly Val Pro Gly Val Gly Val
                405                 410                 415
Pro Gly Val Gly Val Pro Gly Val Gly Val Pro Gly Val Gly Val Pro
            420                 425                 430
Gly Val Gly Val Pro Gly Val Gly Val Pro Gly Val Gly Val Pro Gly
        435                 440                 445
Val Gly Val Pro Gly Val Gly Val Pro Gly Val Gly Val Pro Gly Val
    450                 455                 460
Gly Val Pro Gly Val Gly Val Pro Gly Val Gly Val Pro Gly Val Gly
465                 470                 475                 480
Val Pro Gly Val Gly Val Pro Gly Val Gly Val Pro Gly Val Gly Val
                485                 490                 495
Pro Gly Val Gly Val Pro Gly Val Gly Val Pro Gly Val Gly Val Pro
            500                 505                 510
Gly Val Gly Val Pro Gly Val Gly Val Pro Gly Val Gly Val Pro Gly
        515                 520                 525
Val Gly Val Pro Gly Val Gly Val Pro Gly Val Gly Val Pro Gly Val
    530                 535                 540
Gly Val Pro Gly Val Gly Val Pro Gly Val Gly Val Pro Gly Val Gly
545                 550                 555                 560
Val Pro Gly Val Gly Val Pro Gly Val Gly Val Pro Gly Val Gly Val
                565                 570                 575
Pro Gly Val Gly Val Pro Gly Val Gly Val Pro Gly Val Gly Val Pro
            580                 585                 590
Gly Val Gly Val Pro Gly Val Gly
        595                 600


<210> 3
<211> 110
<212> PRT
<213> Artificial

<220>
<223> Thermosensitive bioelastic polymer sequence

<400> 3
Val Pro Gly Val Gly Val Pro Gly Val Gly Val Pro Gly Val Gly Val
1               5                   10                  15
Pro Gly Val Gly Val Pro Gly Val Gly Val Pro Gly Val Gly Val Pro
            20                  25                  30
Gly Lys Gly Val Pro Gly Lys Gly Val Pro Gly Lys Gly Val Pro Gly
        35                  40                  45
Lys Gly Val Pro Gly Lys Gly Val Pro Gly Lys Gly Val Pro Gly Lys
    50                  55                  60
Gly Val Pro Gly Lys Gly Val Pro Gly Lys Gly Val Pro Gly Lys Gly
65                  70                  75                  80
Val Pro Gly Lys Gly Val Pro Gly Lys Gly Val Pro Gly Lys Gly Val
                85                  90                  95
Pro Gly Lys Gly Val Pro Gly Lys Gly Val Pro Gly Lys Gly
            100                 105                 110


<210> 4

<211> 5
<212> PRT
<213> Artificial

<220>
<223> Bioelastic polymer unit sequence

<220>
<221> MISC_FEATURE
<222> (4)..(4)
<223> Xaa can be any naturally occurring amino acid other than proline

<400> 4
Val Pro Gly Xaa Gly
1               5


<210> 5
<211> 40
<212> PRT
<213> Artificial

<220>
<223> Bioelastic polymer sequence

<400> 5
Val Pro Gly Ala Gly Val Pro Gly Ala Gly Val Pro Gly Ala Gly Val
1               5                   10                  15
Pro Gly Ala Gly Val Pro Gly Ala Gly Val Pro Gly Val Gly Val Pro
            20                  25                  30
Gly Gly Gly Val Pro Gly Gly Gly
        35                  40


<210> 6
<211> 4
<212> PRT
<213> Artificial

<220>
<223> Bioelastic polymer unit sequence

<400> 6
Val Pro Gly Gly
1


<210> 7
<211> 4
<212> PRT
<213> Artificial

<220>
<223> Bioelastic polymer unit sequence

<400> 7
Gly Gly Val Pro
1


<210> 8
<211> 4
<212> PRT
<213> Artificial

<220>
<223> Bioelastic polymer unit sequence

<400> 8
Gly Gly Phe Pro
1

<210> 9
<211> 4
<212> PRT
<213> Artificial

<220>
<223> Bioelastic polymer unit sequence

<400> 9
Gly Gly Ala Pro
1

<210> 10
<211> 5
<212> PRT
<213> Artificial

<220>
<223> Bioelastic polymer unit sequence

<400> 10
Val Pro Gly Val Gly
1               5

<210> 11
<211> 5
<212> PRT
<213> Artificial

<220>
<223> Bioelastic polymer unit sequence

<400> 11
Gly Val Gly Val Pro
1               5

<210> 12
<211> 5
<212> PRT
<213> Artificial

<220>
<223> Bioelastic polymer unit sequence

<400> 12
Gly Lys Gly Val Pro
1               5

<210> 13
<211> 5
<212> PRT
<213> Artificial

<220>
<223> Bioelastic polymer unit sequence

<400> 13
Gly Val Gly Phe Pro
1               5

<210> 14
<211> 5
<212> PRT
<213> Artificial

<220>
<223> Bioelastic polymer unit sequence

<400> 14
Gly Phe Gly Phe Pro
1               5


<210> 15
<211> 5
<212> PRT
<213> Artificial

<220>
<223> Bioelastic polymer unit sequence

<400> 15
Gly Glu Gly Val Pro
1               5


<210> 16
<211> 5
<212> PRT
<213> Artificial

<220>
<223> Bioelastic polymer unit sequence

<400> 16
Gly Phe Gly Val Pro
1               5


<210> 17
<211> 5
<212> PRT
<213> Artificial

<220>
<223> Bioelastic polymer unit sequence

<400> 17
Gly Val Gly Ile Pro
1               5


<210> 18
<211> 800
<212> PRT
<213> Artificial

<220>
<223> Thermosensitive bioelastic polymer sequence

<400> 18
Val Pro Gly Val Gly Val Pro Gly Ala Gly Val Pro Gly Ala Gly Val
1               5                   10                  15
Pro Gly Ala Gly Val Pro Gly Ala Gly Val Pro Gly Ala Gly Val Pro
            20                  25                  30
Gly Ala Gly Val Pro Gly Ala Gly Val Pro Gly Ala Gly Val Pro Gly

35                  40                  45
Gly Gly Val Pro Gly Gly Gly Val Pro Gly Gly Gly Val Pro Gly Gly
        50                  55                  60
Gly Val Pro Gly Gly Gly Val Pro Gly Gly Gly Val Pro Gly Gly Gly
65                  70                  75                  80
Val Pro Gly Val Gly Val Pro Gly Ala Gly Val Pro Gly Ala Gly Val
                85                  90                  95
Pro Gly Ala Gly Val Pro Gly Ala Gly Val Pro Gly Ala Gly Val Pro
            100                 105                 110
Gly Ala Gly Val Pro Gly Ala Gly Val Pro Gly Ala Gly Val Pro Gly
        115                 120                 125
Gly Gly Val Pro Gly Gly Gly Val Pro Gly Gly Gly Val Pro Gly Gly
    130                 135                 140
Gly Val Pro Gly Gly Gly Val Pro Gly Gly Gly Val Pro Gly Gly Gly
145                 150                 155                 160
Val Pro Gly Val Gly Val Pro Gly Ala Gly Val Pro Gly Ala Gly Val
                165                 170                 175
Pro Gly Ala Gly Val Pro Gly Ala Gly Val Pro Gly Ala Gly Val Pro
            180                 185                 190
Gly Ala Gly Val Pro Gly Ala Gly Val Pro Gly Ala Gly Val Pro Gly
        195                 200                 205
Gly Gly Val Pro Gly Gly Gly Val Pro Gly Gly Gly Val Pro Gly Gly
    210                 215                 220
Gly Val Pro Gly Gly Gly Val Pro Gly Gly Gly Val Pro Gly Gly Gly
225                 230                 235                 240
Val Pro Gly Val Gly Val Pro Gly Ala Gly Val Pro Gly Ala Gly Val
                245                 250                 255
Pro Gly Ala Gly Val Pro Gly Ala Gly Val Pro Gly Ala Gly Val Pro
            260                 265                 270
Gly Ala Gly Val Pro Gly Ala Gly Val Pro Gly Ala Gly Val Pro Gly
        275                 280                 285
Gly Gly Val Pro Gly Gly Gly Val Pro Gly Gly Gly Val Pro Gly Gly
    290                 295                 300
Gly Val Pro Gly Gly Gly Val Pro Gly Gly Gly Val Pro Gly Gly Gly
305                 310                 315                 320
Val Pro Gly Val Gly Val Pro Gly Ala Gly Val Pro Gly Ala Gly Val
                325                 330                 335
Pro Gly Ala Gly Val Pro Gly Ala Gly Val Pro Gly Ala Gly Val Pro
            340                 345                 350
Gly Ala Gly Val Pro Gly Ala Gly Val Pro Gly Ala Gly Val Pro Gly
        355                 360                 365
Gly Gly Val Pro Gly Gly Gly Val Pro Gly Gly Gly Val Pro Gly Gly
    370                 375                 380
Gly Val Pro Gly Gly Gly Val Pro Gly Gly Gly Val Pro Gly Gly Gly
385                 390                 395                 400
Val Pro Gly Val Gly Val Pro Gly Ala Gly Val Pro Gly Ala Gly Val
                405                 410                 415
Pro Gly Ala Gly Val Pro Gly Ala Gly Val Pro Gly Ala Gly Val Pro
            420                 425                 430
Gly Ala Gly Val Pro Gly Ala Gly Val Pro Gly Ala Gly Val Pro Gly
        435                 440                 445
Gly Gly Val Pro Gly Gly Gly Val Pro Gly Gly Gly Val Pro Gly Gly
    450                 455                 460
Gly Val Pro Gly Gly Gly Val Pro Gly Gly Gly Val Pro Gly Gly Gly
465                 470                 475                 480
Val Pro Gly Val Gly Val Pro Gly Ala Gly Val Pro Gly Ala Gly Val
                485                 490                 495
Pro Gly Ala Gly Val Pro Gly Ala Gly Val Pro Gly Ala Gly Val Pro
            500                 505                 510
Gly Ala Gly Val Pro Gly Ala Gly Val Pro Gly Ala Gly Val Pro Gly
        515                 520                 525
Gly Gly Val Pro Gly Gly Gly Val Pro Gly Gly Gly Val Pro Gly Gly
    530                 535                 540
Gly Val Pro Gly Gly Gly Val Pro Gly Gly Gly Val Pro Gly Gly Gly
545                 550                 555                 560
Val Pro Gly Val Gly Val Pro Gly Ala Gly Val Pro Gly Ala Gly Val
                565                 570                 575
Pro Gly Ala Gly Val Pro Gly Ala Gly Val Pro Gly Ala Gly Val Pro

580                     585                     590
Gly Ala Gly Val Pro Gly Ala Gly Val Pro Gly Ala Gly Val Pro Gly
        595                     600                     605
Gly Gly Val Pro Gly Gly Gly Val Pro Gly Gly Gly Val Pro Gly Gly
    610                     615                     620
Gly Val Pro Gly Gly Gly Val Pro Gly Gly Gly Val Pro Gly Gly Gly
625                     630                     635                     640
Val Pro Gly Val Gly Val Pro Gly Ala Gly Val Pro Gly Ala Gly Val
                645                     650                     655
Pro Gly Ala Gly Val Pro Gly Ala Gly Val Pro Gly Ala Gly Val Pro
            660                     665                     670
Gly Ala Gly Val Pro Gly Ala Gly Val Pro Gly Ala Gly Val Pro Gly
        675                     680                     685
Gly Gly Val Pro Gly Gly Gly Val Pro Gly Gly Gly Val Pro Gly Gly
    690                     695                     700
Gly Val Pro Gly Gly Gly Val Pro Gly Gly Gly Val Pro Gly Gly Gly
705                     710                     715                     720
Val Pro Gly Val Gly Val Pro Gly Ala Gly Val Pro Gly Ala Gly Val
                725                     730                     735
Pro Gly Ala Gly Val Pro Gly Ala Gly Val Pro Gly Ala Gly Val Pro
            740                     745                     750
Gly Ala Gly Val Pro Gly Ala Gly Val Pro Gly Ala Gly Val Pro Gly
        755                     760                     765
Gly Gly Val Pro Gly Gly Gly Val Pro Gly Gly Gly Val Pro Gly Gly
    770                     775                     780
Gly Val Pro Gly Gly Gly Val Pro Gly Gly Gly Val Pro Gly Gly Gly
785                     790                     795                     800


<210> 19
<211> 560
<212> PRT
<213> Artificial

<220>
<223> Bioelastic polymer sequence

<400> 19
Val Pro Gly Val Gly Val Pro Gly Val Gly Val Pro Gly Val Gly Val
1               5                   10                  15
Pro Gly Val Gly Val Pro Gly Val Gly Val Pro Gly Val Gly Val Pro
            20                  25                  30
Gly Lys Gly Val Pro Gly Val Gly Val Pro Gly Val Gly Val Pro Gly
        35                  40                  45
Val Gly Val Pro Gly Val Gly Val Pro Gly Val Gly Val Pro Gly Val
    50                  55                  60
Gly Val Pro Gly Lys Gly Val Pro Gly Val Gly Val Pro Gly Val Gly
65                  70                  75                  80
Val Pro Gly Val Gly Val Pro Gly Val Gly Val Pro Gly Val Gly Val
                85                  90                  95
Pro Gly Val Gly Val Pro Gly Lys Gly Val Pro Gly Val Gly Val Pro
            100                 105                 110
Gly Val Gly Val Pro Gly Val Gly Val Pro Gly Val Gly Val Pro Gly
        115                 120                 125
Val Gly Val Pro Gly Val Gly Val Pro Gly Lys Gly Val Pro Gly Val
    130                 135                 140
Gly Val Pro Gly Val Gly Val Pro Gly Val Gly Val Pro Gly Val Gly
145                 150                 155                 160
Val Pro Gly Val Gly Val Pro Gly Val Gly Val Pro Gly Lys Gly Val
                165                 170                 175
Pro Gly Val Gly Val Pro Gly Val Gly Val Pro Gly Val Gly Val Pro
            180                 185                 190
Gly Val Gly Val Pro Gly Val Gly Val Pro Gly Val Gly Val Pro Gly
        195                 200                 205
Lys Gly Val Pro Gly Val Gly Val Pro Gly Val Gly Val Pro Gly Val
    210                 215                 220
Gly Val Pro Gly Val Gly Val Pro Gly Val Gly Val Pro Gly Val Gly
225                 230                 235                 240

Val Pro Gly Lys Gly Val Pro Gly Val Gly Val Pro Gly Val Gly Val
                    245                 250                 255
Pro Gly Val Gly Val Pro Gly Val Gly Val Pro Gly Val Gly Val Pro
            260                 265                 270
Gly Val Gly Val Pro Gly Lys Gly Val Pro Gly Val Gly Val Pro Gly
        275                 280                 285
Val Gly Val Pro Gly Val Gly Val Pro Gly Val Gly Val Pro Gly Val
    290                 295                 300
Gly Val Pro Gly Val Gly Val Pro Gly Lys Gly Val Pro Gly Val Gly
305                 310                 315                 320
Val Pro Gly Val Gly Val Pro Gly Val Gly Val Pro Gly Val Gly Val
                    325                 330                 335
Pro Gly Val Gly Val Pro Gly Val Gly Val Pro Gly Lys Gly Val Pro
            340                 345                 350
Gly Val Gly Val Pro Gly Val Gly Val Pro Gly Val Gly Val Pro Gly
        355                 360                 365
Val Gly Val Pro Gly Val Gly Val Pro Gly Val Gly Val Pro Gly Lys
    370                 375                 380
Gly Val Pro Gly Val Gly Val Pro Gly Val Gly Val Pro Gly Val Gly
385                 390                 395                 400
Val Pro Gly Val Gly Val Pro Gly Val Gly Val Pro Gly Val Gly Val
                    405                 410                 415
Pro Gly Lys Gly Val Pro Gly Val Gly Val Pro Gly Val Gly Val Pro
            420                 425                 430
Gly Val Gly Val Pro Gly Val Gly Val Pro Gly Val Gly Val Pro Gly
        435                 440                 445
Val Gly Val Pro Gly Lys Gly Val Pro Gly Val Gly Val Pro Gly Val
    450                 455                 460
Gly Val Pro Gly Val Gly Val Pro Gly Val Gly Val Pro Gly Val Gly
465                 470                 475                 480
Val Pro Gly Val Gly Val Pro Gly Lys Gly Val Pro Gly Val Gly Val
                    485                 490                 495
Pro Gly Val Gly Val Pro Gly Val Gly Val Pro Gly Val Gly Val Pro
            500                 505                 510
Gly Val Gly Val Pro Gly Val Gly Val Pro Gly Lys Gly Val Pro Gly
        515                 520                 525
Val Gly Val Pro Gly Val Gly Val Pro Gly Val Gly Val Pro Gly Val
    530                 535                 540
Gly Val Pro Gly Val Gly Val Pro Gly Val Gly Val Pro Gly Lys Gly
545                 550                 555                 560


<210> 20
<211> 450
<212> PRT
<213> Artificial

<220>
<223> Biolelastic polymer sequence

<400> 20
Val Pro Gly Val Gly Val Pro Gly Val Gly Val Pro Gly Val Gly Val
1               5                   10                  15
Pro Gly Val Gly Val Pro Gly Val Gly Val Pro Gly Gly Gly Val Pro
            20                  25                  30
Gly Gly Gly Val Pro Gly Gly Gly Val Pro Gly Ala Gly Val Pro Gly
        35                  40                  45
Ala Gly Val Pro Gly Val Gly Val Pro Gly Val Gly Val Pro Gly Val
    50                  55                  60
Gly Val Pro Gly Val Gly Val Pro Gly Val Gly Val Pro Gly Gly Gly
65                  70                  75                  80
Val Pro Gly Gly Gly Val Pro Gly Gly Gly Val Pro Gly Ala Gly Val
                85                  90                  95
Pro Gly Ala Gly Val Pro Gly Val Gly Val Pro Gly Val Gly Val Pro
            100                 105                 110
Gly Val Gly Val Pro Gly Val Gly Val Pro Gly Val Gly Val Pro Gly
        115                 120                 125
Gly Gly Val Pro Gly Gly Gly Val Pro Gly Gly Gly Val Pro Gly Ala

130                 135                 140
Gly Val Pro Gly Ala Gly Val Pro Gly Val Gly Val Pro Gly Val Gly
145                 150                 155                 160
Val Pro Gly Val Gly Val Pro Gly Val Gly Val Pro Gly Val Gly Val
                165                 170                 175
Pro Gly Gly Gly Val Pro Gly Gly Gly Val Pro Gly Gly Gly Val Pro
            180                 185                 190
Gly Ala Gly Val Pro Gly Ala Gly Val Pro Gly Val Gly Val Pro Gly
        195                 200                 205
Val Gly Val Pro Gly Val Gly Val Pro Gly Val Gly Val Pro Gly Val
    210                 215                 220
Gly Val Pro Gly Gly Gly Val Pro Gly Gly Gly Val Pro Gly Gly Gly
225                 230                 235                 240
Val Pro Gly Ala Gly Val Pro Gly Ala Gly Val Pro Gly Val Gly Val
                245                 250                 255
Pro Gly Val Gly Val Pro Gly Val Gly Val Pro Gly Val Gly Val Pro
            260                 265                 270
Gly Val Gly Val Pro Gly Gly Gly Val Pro Gly Gly Gly Val Pro Gly
        275                 280                 285
Gly Gly Val Pro Gly Ala Gly Val Pro Gly Ala Gly Val Pro Gly Val
    290                 295                 300
Gly Val Pro Gly Val Gly Val Pro Gly Val Gly Val Pro Gly Val Gly
305                 310                 315                 320
Val Pro Gly Val Gly Val Pro Gly Gly Gly Val Pro Gly Gly Gly Val
                325                 330                 335
Pro Gly Gly Gly Val Pro Gly Ala Gly Val Pro Gly Ala Gly Val Pro
            340                 345                 350
Gly Val Gly Val Pro Gly Val Gly Val Pro Gly Val Gly Val Pro Gly
        355                 360                 365
Val Gly Val Pro Gly Val Gly Val Pro Gly Gly Gly Val Pro Gly Gly
    370                 375                 380
Gly Val Pro Gly Gly Gly Val Pro Gly Ala Gly Val Pro Gly Ala Gly
385                 390                 395                 400
Val Pro Gly Val Gly Val Pro Gly Val Gly Val Pro Gly Val Gly Val
                405                 410                 415
Pro Gly Val Gly Val Pro Gly Val Gly Val Pro Gly Gly Gly Val Pro
            420                 425                 430
Gly Gly Gly Val Pro Gly Gly Gly Val Pro Gly Ala Gly Val Pro Gly
        435                 440                 445
Ala Gly
    450

<210> 21
<211> 150
<212> PRT
<213> Artificial

<220>
<223> Bioelastic polymer sequence

<400> 21
Val Pro Gly Val Gly Val Pro Gly Val Gly Val Pro Gly Val Gly Val
1               5                   10                  15
Pro Gly Val Gly Val Pro Gly Val Gly Val Pro Gly Val Gly Val Pro
            20                  25                  30
Gly Val Gly Val Pro Gly Val Gly Val Pro Gly Val Gly Val Pro Gly
        35                  40                  45
Val Gly Val Pro Gly Val Gly Val Pro Gly Val Gly Val Pro Gly Val
    50                  55                  60
Gly Val Pro Gly Val Gly Val Pro Gly Val Gly Val Pro Gly Val Gly
65                  70                  75                  80
Val Pro Gly Val Gly Val Pro Gly Val Gly Val Pro Gly Val Gly Val
                85                  90                  95
Pro Gly Val Gly Val Pro Gly Val Gly Val Pro Gly Val Gly Val Pro
            100                 105                 110
Gly Val Gly Val Pro Gly Val Gly Val Pro Gly Val Gly Val Pro Gly
        115                 120                 125

Val Gly Val Pro Gly Val Gly Val Pro Gly Val Gly Val Pro Gly Val
    130                 135                 140
Gly Val Pro Gly Val Gly
145                 150

<210> 22
<211> 945
<212> DNA
<213> Artificial

<220>
<223> Elastin-like polypeptide coding sequence

<220>
<221> CDS
<222> (1)..(942)

<400> 22
atg agc ggg ccg ggc gtg ggt gtt ccg ggc gta ggt gtc cca ggt gtg      48
Met Ser Gly Pro Gly Val Gly Val Pro Gly Val Gly Val Pro Gly Val
1               5                   10                  15
ggc gta ccg ggc gtt ggt gtt cct ggt gtc ggc gtg ccg ggc gtg ggt      96
Gly Val Pro Gly Val Gly Val Pro Gly Val Gly Val Pro Gly Val Gly
            20                  25                  30
gtt ccg ggc gta ggt gtc cca ggt gtg ggc gta ccg ggc gtt ggt gtt      144
Val Pro Gly Val Gly Val Pro Gly Val Gly Val Pro Gly Val Gly Val
        35                  40                  45
cct ggt gtc ggc gtg ccg ggc gtg ggt gtt ccg ggc gta ggt gtc cca      192
Pro Gly Val Gly Val Pro Gly Val Gly Val Pro Gly Val Gly Val Pro
    50                  55                  60
ggt gtg ggc gta ccg ggc gtt ggt gtt cct ggt gtc ggc gtg ccg ggc      240
Gly Val Gly Val Pro Gly Val Gly Val Pro Gly Val Gly Val Pro Gly
65                  70                  75                  80
gtg ggt gtt ccg ggc gta ggt gtc cca ggt gtg ggc gta ccg ggc gtt      288
Val Gly Val Pro Gly Val Gly Val Pro Gly Val Gly Val Pro Gly Val
                85                  90                  95
ggt gtt cct ggt gtc ggc gtg ccg ggc gtg ggt gtt ccg ggc gta ggt      336
Gly Val Pro Gly Val Gly Val Pro Gly Val Gly Val Pro Gly Val Gly
            100                 105                 110
gtc cca ggt gtg ggc gta ccg ggc gtt ggt gtt cct ggt gtc ggc gtg      384
Val Pro Gly Val Gly Val Pro Gly Val Gly Val Pro Gly Val Gly Val
        115                 120                 125
ccg ggc gtg ggt gtt ccg ggc gta ggt gtc cca ggt gtg ggc gta ccg      432
Pro Gly Val Gly Val Pro Gly Val Gly Val Pro Gly Val Gly Val Pro
    130                 135                 140
ggc gtt ggt gtt cct ggt gtc ggc gtg ccg ggc ggg ccg ggc ggc agc      480
Gly Val Gly Val Pro Gly Val Gly Val Pro Gly Gly Pro Gly Gly Ser
145                 150                 155                 160
ggc ggc agc gga tcc ggg aga aaa tcc agc aag atg caa gcc ttc aga      528
Gly Gly Ser Gly Ser Gly Arg Lys Ser Ser Lys Met Gln Ala Phe Arg
                165                 170                 175
atc tgg gat gtt aac cag aag acc ttc tat ctg agg aac aac caa cta      576
Ile Trp Asp Val Asn Gln Lys Thr Phe Tyr Leu Arg Asn Asn Gln Leu
            180                 185                 190
gtt gct gga tac ttg caa gga cca aat gtc aat tta gaa gaa aag ata      624
Val Ala Gly Tyr Leu Gln Gly Pro Asn Val Asn Leu Glu Glu Lys Ile
        195                 200                 205
gat gtg gta ccc att gag cct cat gct ctg ttc ttg gga atc cat gga      672
Asp Val Val Pro Ile Glu Pro His Ala Leu Phe Leu Gly Ile His Gly
    210                 215                 220
ggg aag atg tgc ctg tcc tgt gtc aag tct ggt gat gag acc aga ctc      720
Gly Lys Met Cys Leu Ser Cys Val Lys Ser Gly Asp Glu Thr Arg Leu
225                 230                 235                 240
cag ctg gag gca gtt aac atc act gac ctg agc gag aac aga aag cag      768
Gln Leu Glu Ala Val Asn Ile Thr Asp Leu Ser Glu Asn Arg Lys Gln
                245                 250                 255
gac aag cgc ttc gcc ttc atc cgc tca gac agc ggc ccc acc acc agt      816

Asp Lys Arg Phe Ala Phe Ile Arg Ser Asp Ser Gly Pro Thr Thr Ser
            260                 265                 270
ttt gag tct gcc gcc tgc ccc ggt tgg ttc ctc tgc aca gcg atg gaa 864
Phe Glu Ser Ala Ala Cys Pro Gly Trp Phe Leu Cys Thr Ala Met Glu
        275                 280                 285
gct gac cag ccc gtc agc ctc acc aat atg cct gac gaa ggc gtc atg 912
Ala Asp Gln Pro Val Ser Leu Thr Asn Met Pro Asp Glu Gly Val Met
    290                 295                 300
gtc acc aaa ttc tac ttc cag gag gac gag tag 945
Val Thr Lys Phe Tyr Phe Gln Glu Asp Glu
305                 310

<210> 23
<211> 314
<212> PRT
<213> Artificial

<220>
<223> Synthetic Construct

<400> 23
Met Ser Gly Pro Gly Val Gly Val Pro Gly Val Gly Val Pro Gly Val
1               5                   10                  15
Gly Val Pro Gly Val Gly Val Pro Gly Val Gly Val Pro Gly Val Gly
            20                  25                  30
Val Pro Gly Val Gly Val Pro Gly Val Gly Val Pro Gly Val Gly Val
        35                  40                  45
Pro Gly Val Gly Val Pro Gly Val Gly Val Pro Gly Val Gly Val Pro
    50                  55                  60
Gly Val Gly Val Pro Gly Val Gly Val Pro Gly Val Gly Val Pro Gly
65                  70                  75                  80
Val Gly Val Pro Gly Val Gly Val Pro Gly Val Gly Val Pro Gly Val
                85                  90                  95
Gly Val Pro Gly Val Gly Val Pro Gly Val Gly Val Pro Gly Val Gly
            100                 105                 110
Val Pro Gly Val Gly Val Pro Gly Val Gly Val Pro Gly Val Gly Val
        115                 120                 125
Pro Gly Val Gly Val Pro Gly Val Gly Val Pro Gly Val Gly Val Pro
    130                 135                 140
Gly Val Gly Val Pro Gly Val Gly Val Pro Gly Gly Pro Gly Gly Ser
145                 150                 155                 160
Gly Gly Ser Gly Ser Gly Arg Lys Ser Ser Lys Met Gln Ala Phe Arg
                165                 170                 175
Ile Trp Asp Val Asn Gln Lys Thr Phe Tyr Leu Arg Asn Asn Gln Leu
            180                 185                 190
Val Ala Gly Tyr Leu Gln Gly Pro Asn Val Asn Leu Glu Glu Lys Ile
        195                 200                 205
Asp Val Val Pro Ile Glu Pro His Ala Leu Phe Leu Gly Ile His Gly
    210                 215                 220
Gly Lys Met Cys Leu Ser Cys Val Lys Ser Gly Asp Glu Thr Arg Leu
225                 230                 235                 240
Gln Leu Glu Ala Val Asn Ile Thr Asp Leu Ser Glu Asn Arg Lys Gln
                245                 250                 255
Asp Lys Arg Phe Ala Phe Ile Arg Ser Asp Ser Gly Pro Thr Thr Ser
            260                 265                 270
Phe Glu Ser Ala Ala Cys Pro Gly Trp Phe Leu Cys Thr Ala Met Glu
        275                 280                 285
Ala Asp Gln Pro Val Ser Leu Thr Asn Met Pro Asp Glu Gly Val Met
    290                 295                 300
Val Thr Lys Phe Tyr Phe Gln Glu Asp Glu
305                 310

<210> 24
<211> 1845
<212> DNA
<213> Artificial

<220>
<223> Elastin-like polypeptide coding sequence

<220>
<221> CDS
<222> (1)..(1842)

<400> 24
atg agc ggg ccg ggc gtg ggt gtt ccg ggc gtg ggt gtt ccg ggt ggc      48
Met Ser Gly Pro Gly Val Gly Val Pro Gly Val Gly Val Pro Gly Gly
1               5                   10                  15
ggt gtg ccg ggc gca ggt gtt cct ggt gta ggt gtg ccg ggt gtt ggt      96
Gly Val Pro Gly Ala Gly Val Pro Gly Val Gly Val Pro Gly Val Gly
            20                  25                  30
gtg ccg ggt gtt ggt gta cca ggt ggc ggt gtt ccg ggt gca ggc gtt      144
Val Pro Gly Val Gly Val Pro Gly Gly Gly Val Pro Gly Ala Gly Val
        35                  40                  45
ccg ggt ggc ggt gtg ccg ggc gtg ggt gtt ccg ggc gtg ggt gtt ccg      192
Pro Gly Gly Gly Val Pro Gly Val Gly Val Pro Gly Val Gly Val Pro
    50                  55                  60
ggt ggc ggt gtg ccg ggc gca ggt gtt cct ggt gta ggt gtg ccg ggt      240
Gly Gly Gly Val Pro Gly Ala Gly Val Pro Gly Val Gly Val Pro Gly
65                  70                  75                  80
gtt ggt gtg ccg ggt gtt ggt gta cca ggt ggc ggt gtt ccg ggt gca      288
Val Gly Val Pro Gly Val Gly Val Pro Gly Gly Gly Val Pro Gly Ala
                85                  90                  95
ggc gtt ccg ggt ggc ggt gtg ccg ggc gtg ggt gtt ccg ggc gtg ggt      336
Gly Val Pro Gly Gly Gly Val Pro Gly Val Gly Val Pro Gly Val Gly
            100                 105                 110
gtt ccg ggt ggc ggt gtg ccg ggc gca ggt gtt cct ggt gta ggt gtg      384
Val Pro Gly Gly Gly Val Pro Gly Ala Gly Val Pro Gly Val Gly Val
        115                 120                 125
ccg ggt gtt ggt gtg ccg ggt gtt ggt gta cca ggt ggc ggt gtt ccg      432
Pro Gly Val Gly Val Pro Gly Val Gly Val Pro Gly Gly Gly Val Pro
    130                 135                 140
ggt gca ggc gtt ccg ggt ggc ggt gtg ccg ggc gtg ggt gtt ccg ggc      480
Gly Ala Gly Val Pro Gly Gly Gly Val Pro Gly Val Gly Val Pro Gly
145                 150                 155                 160
gtg ggt gtt ccg ggt ggc ggt gtg ccg ggc gca ggt gtt cct ggt gta      528
Val Gly Val Pro Gly Gly Gly Val Pro Gly Ala Gly Val Pro Gly Val
                165                 170                 175
ggt gtg ccg ggt gtt ggt gtg ccg ggt gtt ggt gta cca ggt ggc ggt      576
Gly Val Pro Gly Val Gly Val Pro Gly Val Gly Val Pro Gly Gly Gly
            180                 185                 190
gtt ccg ggt gca ggc gtt ccg ggt ggc ggt gtg ccg ggc gtg ggt gtt      624
Val Pro Gly Ala Gly Val Pro Gly Gly Gly Val Pro Gly Val Gly Val
        195                 200                 205
ccg ggc gtg ggt gtt ccg ggt ggc ggt gtg ccg ggc gca ggt gtt cct      672
Pro Gly Val Gly Val Pro Gly Gly Gly Val Pro Gly Ala Gly Val Pro
    210                 215                 220
ggt gta ggt gtg ccg ggt gtt ggt gtg ccg ggt gtt ggt gta cca ggt      720
Gly Val Gly Val Pro Gly Val Gly Val Pro Gly Val Gly Val Pro Gly
225                 230                 235                 240
ggc ggt gtt ccg ggt gca ggc gtt ccg ggt ggc ggt gtg ccg ggc gtg      768
Gly Gly Val Pro Gly Ala Gly Val Pro Gly Gly Gly Val Pro Gly Val
                245                 250                 255
ggt gtt ccg ggc gtg ggt gtt ccg ggt ggc ggt gtg ccg ggc gca ggt      816
Gly Val Pro Gly Val Gly Val Pro Gly Gly Gly Val Pro Gly Ala Gly
            260                 265                 270
gtt cct ggt gta ggt gtg ccg ggt gtt ggt gtg ccg ggt gtt ggt gta      864
Val Pro Gly Val Gly Val Pro Gly Val Gly Val Pro Gly Val Gly Val
        275                 280                 285
cca ggt ggc ggt gtt ccg ggt gca ggc gtt ccg ggt ggc ggt gtg ccg      912
Pro Gly Gly Gly Val Pro Gly Ala Gly Val Pro Gly Gly Gly Val Pro
    290                 295                 300
ggc gtg ggt gtt ccg ggc gtg ggt gtt ccg ggt ggc ggt gtg ccg ggc      960
Gly Val Gly Val Pro Gly Val Gly Val Pro Gly Gly Gly Val Pro Gly

305                 310                 315                 320
gca ggt gtt cct ggt gta ggt gtg ccg ggt gtt ggt gtg ccg ggt gtt      1008
Ala Gly Val Pro Gly Val Gly Val Pro Gly Val Gly Val Pro Gly Val
                325                 330                 335
ggt gta cca ggt ggc ggt gtt ccg ggt gca ggc gtt ccg ggt ggc ggt      1056
Gly Val Pro Gly Gly Gly Val Pro Gly Ala Gly Val Pro Gly Gly Gly
            340                 345                 350
gtg ccg ggc gtg ggt gtt ccg ggc gtg ggt gtt ccg ggt ggc ggt gtg      1104
Val Pro Gly Val Gly Val Pro Gly Val Gly Val Pro Gly Gly Gly Val
        355                 360                 365
ccg ggc gca ggt gtt cct ggt gta ggt gtg ccg ggt gtt ggt gtg ccg      1152
Pro Gly Ala Gly Val Pro Gly Val Gly Val Pro Gly Val Gly Val Pro
    370                 375                 380
ggt gtt ggt gta cca ggt ggc ggt gtt ccg ggt gca ggc gtt ccg ggt      1200
Gly Val Gly Val Pro Gly Gly Gly Val Pro Gly Ala Gly Val Pro Gly
385                 390                 395                 400
ggc ggt gtg ccg ggc gtg ggt gtt ccg ggc gtg ggt gtt ccg ggt ggc      1248
Gly Gly Val Pro Gly Val Gly Val Pro Gly Val Gly Val Pro Gly Gly
                405                 410                 415
ggt gtg ccg ggc gca ggt gtt cct ggt gta ggt gtg ccg ggt gtt ggt      1296
Gly Val Pro Gly Ala Gly Val Pro Gly Val Gly Val Pro Gly Val Gly
            420                 425                 430
gtg ccg ggt gtt ggt gta cca ggt ggc ggt gtt ccg ggt gca ggc gtt      1344
Val Pro Gly Val Gly Val Pro Gly Gly Gly Val Pro Gly Ala Gly Val
        435                 440                 445
ccg ggt ggc ggt gtg ccg ggc ggg ccg ggc ggc agc ggc ggc agc gga      1392
Pro Gly Gly Gly Val Pro Gly Gly Pro Gly Gly Ser Gly Gly Ser Gly
    450                 455                 460
tcc ggg aga aaa tcc agc aag atg caa gcc ttc aga atc tgg gat gtt      1440
Ser Gly Arg Lys Ser Ser Lys Met Gln Ala Phe Arg Ile Trp Asp Val
465                 470                 475                 480
aac cag aag acc ttc tat ctg agg aac aac caa cta gtt gct gga tac      1488
Asn Gln Lys Thr Phe Tyr Leu Arg Asn Asn Gln Leu Val Ala Gly Tyr
                485                 490                 495
ttg caa gga cca aat gtc aat tta gaa gaa aag ata gat gtg gta ccc      1536
Leu Gln Gly Pro Asn Val Asn Leu Glu Glu Lys Ile Asp Val Val Pro
            500                 505                 510
att gag cct cat gct ctg ttc ttg gga atc cat gga ggg aag atg tgc      1584
Ile Glu Pro His Ala Leu Phe Leu Gly Ile His Gly Gly Lys Met Cys
        515                 520                 525
ctg tcc tgt gtc aag tct ggt gat gag acc aga ctc cag ctg gag gca      1632
Leu Ser Cys Val Lys Ser Gly Asp Glu Thr Arg Leu Gln Leu Glu Ala
    530                 535                 540
gtt aac atc act gac ctg agc gag aac aga aag cag gac aag cgc ttc      1680
Val Asn Ile Thr Asp Leu Ser Glu Asn Arg Lys Gln Asp Lys Arg Phe
545                 550                 555                 560
gcc ttc atc cgc tca gac agc ggc ccc acc acc agt ttt gag tct gcc      1728
Ala Phe Ile Arg Ser Asp Ser Gly Pro Thr Thr Ser Phe Glu Ser Ala
                565                 570                 575
gcc tgc ccc ggt tgg ttc ctc tgc aca gcg atg gaa gct gac cag ccc      1776
Ala Cys Pro Gly Trp Phe Leu Cys Thr Ala Met Glu Ala Asp Gln Pro
            580                 585                 590
gtc agc ctc acc aat atg cct gac gaa ggc gtc atg gtc acc aaa ttc      1824
Val Ser Leu Thr Asn Met Pro Asp Glu Gly Val Met Val Thr Lys Phe
        595                 600                 605
tac ttc cag gag gac gag tag                                          1845
Tyr Phe Gln Glu Asp Glu
    610


<210> 25
<211> 614
<212> PRT
<213> Artificial

<220>
<223> Synthetic Construct

<400> 25
Met Ser Gly Pro Gly Val Gly Val Pro Gly Val Gly Val Pro Gly Gly
1               5                   10                  15
Gly Val Pro Gly Ala Gly Val Pro Gly Val Gly Val Pro Gly Val Gly
            20                  25                  30
Val Pro Gly Val Gly Val Pro Gly Gly Gly Val Pro Gly Ala Gly Val
        35                  40                  45
Pro Gly Gly Gly Val Pro Gly Val Gly Val Pro Gly Val Gly Val Pro
    50                  55                  60
Gly Gly Gly Val Pro Gly Ala Gly Val Pro Gly Val Gly Val Pro Gly
65                  70                  75                  80
Val Gly Val Pro Gly Val Gly Val Pro Gly Gly Gly Val Pro Gly Ala
                85                  90                  95
Gly Val Pro Gly Gly Gly Val Pro Gly Val Gly Val Pro Gly Val Gly
            100                 105                 110
Val Pro Gly Gly Gly Val Pro Gly Ala Gly Val Pro Gly Val Gly Val
        115                 120                 125
Pro Gly Val Gly Val Pro Gly Val Gly Val Pro Gly Gly Gly Val Pro
    130                 135                 140
Gly Ala Gly Val Pro Gly Gly Gly Val Pro Gly Val Gly Val Pro Gly
145                 150                 155                 160
Val Gly Val Pro Gly Gly Gly Val Pro Gly Ala Gly Val Pro Gly Val
                165                 170                 175
Gly Val Pro Gly Val Gly Val Pro Gly Val Gly Val Pro Gly Gly Gly
            180                 185                 190
Val Pro Gly Ala Gly Val Pro Gly Gly Gly Val Pro Gly Val Gly Val
        195                 200                 205
Pro Gly Val Gly Val Pro Gly Gly Gly Val Pro Gly Ala Gly Val Pro
    210                 215                 220
Gly Val Gly Val Pro Gly Val Gly Val Pro Gly Val Gly Val Pro Gly
225                 230                 235                 240
Gly Gly Val Pro Gly Ala Gly Val Pro Gly Gly Gly Val Pro Gly Val
                245                 250                 255
Gly Val Pro Gly Val Gly Val Pro Gly Gly Gly Val Pro Gly Ala Gly
            260                 265                 270
Val Pro Gly Val Gly Val Pro Gly Val Gly Val Pro Gly Val Gly Val
        275                 280                 285
Pro Gly Gly Gly Val Pro Gly Ala Gly Val Pro Gly Gly Gly Val Pro
    290                 295                 300
Gly Val Gly Val Pro Gly Val Gly Val Pro Gly Gly Gly Val Pro Gly
305                 310                 315                 320
Ala Gly Val Pro Gly Val Gly Val Pro Gly Val Gly Val Pro Gly Val
                325                 330                 335
Gly Val Pro Gly Gly Gly Val Pro Gly Ala Gly Val Pro Gly Gly Gly
            340                 345                 350
Val Pro Gly Val Gly Val Pro Gly Val Gly Val Pro Gly Gly Gly Val
        355                 360                 365
Pro Gly Ala Gly Val Pro Gly Val Gly Val Pro Gly Val Gly Val Pro
    370                 375                 380
Gly Val Gly Val Pro Gly Gly Gly Val Pro Gly Ala Gly Val Pro Gly
385                 390                 395                 400
Gly Gly Val Pro Gly Val Gly Val Pro Gly Val Gly Val Pro Gly Gly
                405                 410                 415
Gly Val Pro Gly Ala Gly Val Pro Gly Val Gly Val Pro Gly Val Gly
            420                 425                 430
Val Pro Gly Val Gly Val Pro Gly Gly Gly Val Pro Gly Ala Gly Val
        435                 440                 445
Pro Gly Gly Gly Val Pro Gly Gly Pro Gly Gly Ser Gly Gly Ser Gly
    450                 455                 460
Ser Gly Arg Lys Ser Ser Lys Met Gln Ala Phe Arg Ile Trp Asp Val
465                 470                 475                 480
Asn Gln Lys Thr Phe Tyr Leu Arg Asn Asn Gln Leu Val Ala Gly Tyr
                485                 490                 495
Leu Gln Gly Pro Asn Val Asn Leu Glu Glu Lys Ile Asp Val Val Pro
            500                 505                 510
Ile Glu Pro His Ala Leu Phe Leu Gly Ile His Gly Gly Lys Met Cys
        515                 520                 525
Leu Ser Cys Val Lys Ser Gly Asp Glu Thr Arg Leu Gln Leu Glu Ala

530                 535                 540
Val Asn Ile Thr Asp Leu Ser Glu Asn Arg Lys Gln Asp Lys Arg Phe
545                 550                 555                 560
Ala Phe Ile Arg Ser Asp Ser Gly Pro Thr Thr Ser Phe Glu Ser Ala
                565                 570                 575
Ala Cys Pro Gly Trp Phe Leu Cys Thr Ala Met Glu Ala Asp Gln Pro
            580                 585                 590
Val Ser Leu Thr Asn Met Pro Asp Glu Gly Val Met Val Thr Lys Phe
        595                 600                 605
Tyr Phe Gln Glu Asp Glu
    610

## Description

This invention was made with Government support under Grant Nos. AR047442 and EB002263 from the National Institutes of Health. The Government has certain rights to this invention.

### Related Applications

This application claims the benefit of United States Provisional Patent Application Serial No. 60/693,966, filed June 24, 2005, the disclosure of which is incorporated by reference herein in its entirety.

### Field of the Invention

The present invention concerns methods and compositions for the controlled released delivery of pharmaceutical compounds.

### Background of the Invention

Osteoarthritis (OA) is a degenerative joint disease that affects more patients than any other musculoskeletal disorder. It accounts for over 4 million hospitalizations every year and its prevalence is estimated to increase by over 50% in the next 20 years. OA affects individual joints and was historically considered to arise from a "wear and tear" due to joint loading. OA is now understood to arise from multiple causes, including biochemical, genetic and biomechanical factors, that interact and promote the progression of joint disease. While surgical options for joint re-alignment (osteotomy), prosthetic replacement (arthroplasty), or fusion (arthrodesis) are available to patients with advanced joint degeneration, only a small fraction of patients need operative treatments. Hence, there is a great and increasing need to provide non-surgical treatments that are capable of both alleviating patient symptoms and modifying the course of disease progression.

The most widely available treatments for the patient with early to moderate joint disease are the chronic use of NSAIDs. Although these drugs provide effectiveness in pain relief, they are associated with frequent adverse conditions including gastrointestinal bleeding and ulceration. Additionally, NSAIDs are reported to inhibit cell biosynthesis which may compromise the long-term intrinsic repair process in joint degeneration. More recently protein drugs with the ability to modify the disease state of OA have been identified. These protein drugs include TNF-a inhibitors and IL-1 inhibitors have shown significant promise both in preclinical and clinical studies in preventing the onset and retarding the progression of the disease.

Since OA is a disease that is localized to a few joints at a time, localized delivery of the therapeutic is highly preferred. One such technique is the administration of the drug directly into the affected joint cavity, i.e. intra-articular injection, which is currently recommended for corticosteroids and hyaluronan solutions in treating OA. Although the intra-articular mechanism of drug delivery is attractive to the patient and clinician alike, it is compromised by the presence of a highly efficient lymphatic system that rapidly clears molecules from the synovial cavity. Consequently, the therapeutic drug has to be administered frequently or at high concentrations to be effective. This, in turn, may be costly and result in adverse side effects and high levels of patient discomfort. Controlled drug delivery systems have been sought-after to overcome these challenges.

US Patent No. 6,328,996 to Urry describes bioelastomeric drug delivery systems, but does not suggest them for administration directly into a region of interest.

### Summary of the Invention

An objective of the present invention is to provide a thermally responsive biocompatible polymer for the sustained delivery of drugs such as IL-1 receptor antagonist. The delivery system in the present invention utilizes the unique rapid aggregation and slow disaggregation properties of thermally responsive biopolymers to deliver protein drugs directly to a pathologic site, such as an OA affected joint.

The present invention provides a method for delivering a drug depot of a compound of interest to a selected region in a subject. The method comprises administering a composition directly to said region of interest, the composition comprising said compound to be delivered and a polymer that undergoes an inverse temperature phase transition, so that a sustained release of said compound of interest at said selected region is provided.

In some embodiments the present invention provides a method for delivering a drug depot of a compound of interest to a selected region in a subject (*e.g.,* a joint or synovial joint). The method comprises administering a composition to region of interest (*e.g.,* directly, such as by injection in or to the region of interest). The composition comprises the compound to be delivered and a polymer that undergoes an inverse temperature phase transition. The polymer has a transition temperature (Tₜ) less than the body temperature of the subject (*e.g.,* less than 37 °C). The composition or conjugate aggregates in the region of interest and then gradually disaggregates, providing a sustained or controlled release of the compound of interest at the selected region.

In some embodiments the present invention provides a method for delivering a drug depot of a compound of interest to a selected region in a subject, said method comprising: administering a composition directly to said region of interest, said composition comprising said compound to be delivered and a polymer that undergoes an inverse temperature phase transition; wherein said polymer has a transition temperature (Tₜ) less than the body temperature of said subject; so that said composition (or said conjugate) separates from solution in said region of interest to form a bulk aggregate, and then gradually separates from bulk aggregate to go back to solution, providing a sustained release of said compound of interest at said selected region (or stated differently, so that said conjugate separates from solution in said region of interest and then gradually goes back to solution phase, providing a sustained release of said compound of interest at said selected region.).

In some embodiments the composition comprises the compound to be delivered conjugated to the polymer; in other embodiments the composition comprises the compound to be delivered mixed with, but not otherwise conjugated to or chemically coupled to, the polymer.

A further aspect of the invention is a pharmaceutically acceptable composition comprising a therapeutic compound in combination with (*e.g.,* mixed with or conjugated to) a polymer that undergoes an inverse temperature phase transition. Examples of such therapeutic compounds include but are not limited to TNF antibodies, IL-1 antibodies, soluble TNF receptors, soluble IL-1 receptors, TNF receptor antagonists, and IL-1 receptor antagonists, with a particular example being recombinant human IL-1 receptor antagonist and its isoforms.

A further aspect of the invention is an injection device (including syringes and other injection devices) containing a composition as described herein.

A further aspect of the invention is the use of an injection device as described herein for carrying out a method as described herein.

### Brief Description of the Drawings

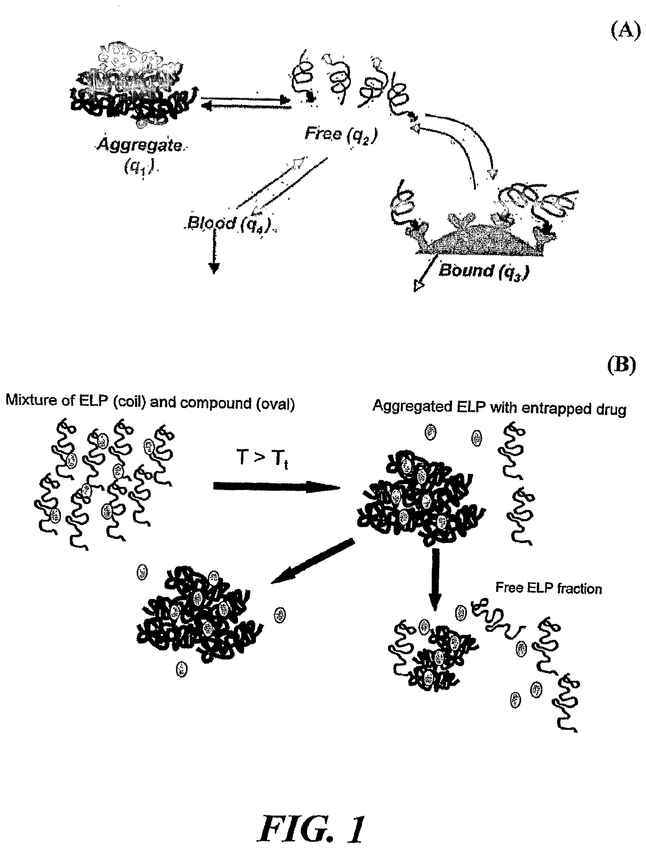

**Figure 1****.** Schematic illustrating examples one embodiment of a of proposed drug delivery system. (a) A compound (drug) attached to a thermosensitive bioelastic polymer (ELP) may be injected into a tissue region (e.g., joint cavity), and the release of the compound occurs over time from aggregate. In its free form, the compound is available to bind to a receptor as shown. The "free" fraction of compound-bioelastic polymer will be cleared from the tissue region (e.g., joint cavity) over time. (b) Alternate example showing a compound initially mixed with a thermosensitive bioelastic polymer during or prior to delivery to a tissue region, promoting entrapment of the compound. The release of the compound occurs over time, with the "free" compound cleared from the tissue region over time.

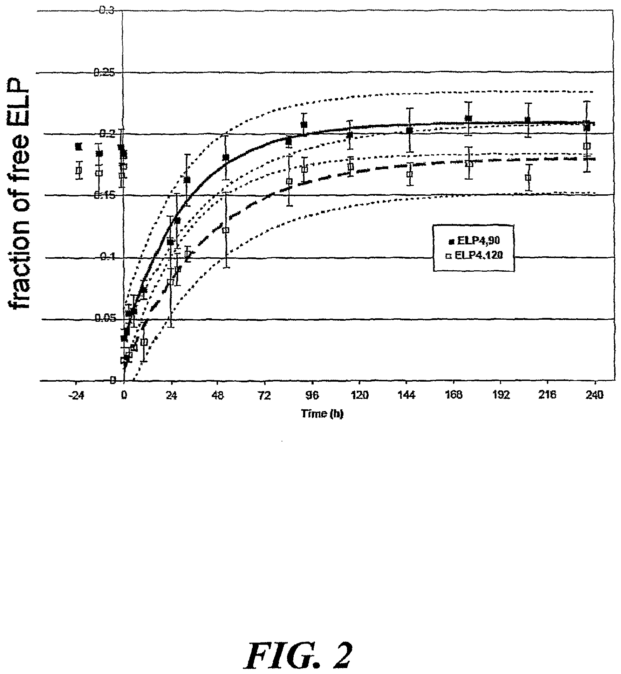

**Figure 2****.** Fraction of ELP detected in supernatant over time, following equilibration of an ELP aggregate at 37°C. Results are shown for ELP4-90 ([(VPGVG)₁₀]₉, **SEQ ID NO:1**) and ELP4-120 ([(VPGVG)₁₀]₁₂, **SEQ ID NO:2**) (mean ± SD, n=4). ELP above its transition temperature was equilibrated at 37°C to promote the inverse temperature phase transition. Supernatant was replaced with fresh PBS at 37°C and ELP concentration in aliquots of supernatant was quantified over time by absorbance at 280 nm. Data were fit to a first-order exponential function (solid line) of the form [ELP]=[ELP]ₛₛ - A exp(-t/τ), to obtain a time constant of equilibration (τ) and a fraction of ELP not contained in aggregate form at equilibrium ([ELP]ₛₛ). For ELP4-90, constants were found to be: [ELP]ₛₛ = 0.21 and τ= 31h. For ELP4-120, constants were found to be: [ELP]ₛₛ = 0.18 and τ= 43h.

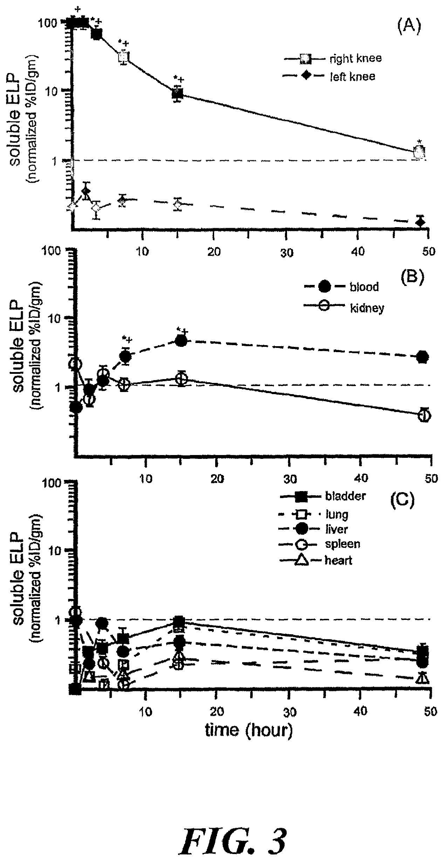

**Figure 3****.** Biodistribution of non-aggregating [¹⁴C]ELP (Tₜ > 50°C) after intra-articular injection into the right knee joint in a rat model. The injected dose (ID/gm) is amount of ¹⁴C recovered from the injected knee compartment (per gram of recovered tissues and fluids) at 10 min post injection. For individual compartments, ¹⁴C (per gram of recovered tissue or fluid) was normalized by this value to determine a %ID/gm for each tissue or fluid. All data expressed as mean ± SE (n=5). (A) * p<0.05, statistically different from time zero; + p<0.05, statistically different from uninjected (left) knee compartment. (B) * p<0.05, statistically different from time zero; + p<0.05, statistically different from blood. (C) The concentration of [¹⁴C]ELP was found to be below 1 %ID/gm for all organs, and these tissues were not found to be statistically different from the uninjected (left) knee at all time points.

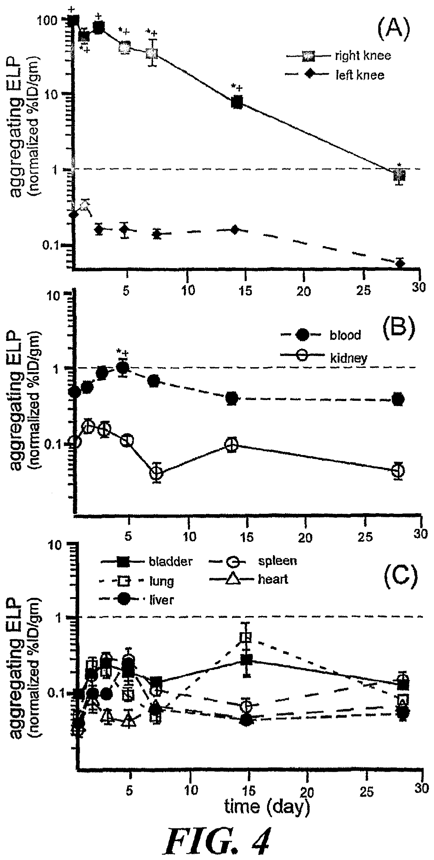

**Figure 4****.** Biodistribution of the aggregating [¹⁴C]ELP (Tₜ < 35°C) after intra-articular injection into the right knee joint of a rat model. The injected dose (ID) is amount of ¹⁴C recovered from the injected knee compartment (per gram of recovered tissues and fluids) at time zero (10 min post injection). For individual compartments, ¹⁴C (per gram of recovered tissue or fluid) was normalized by this value to determine a %ID/gm for each tissue or fluid. All data expressed as mean ± SE (n=5). (A) * p<0.05, statistically different from time zero; + p<0.05, statistically different from uninjected (left) knee compartment. (B) * p<0.05, statistically different from time zero; + p<0.05, statistically different from blood. (C) The concentration of [¹⁴C]ELP was found to be below 1 %ID/gm for all organs, and these tissues were not found to be statistically different from the uninjected (left) knee at all time points.

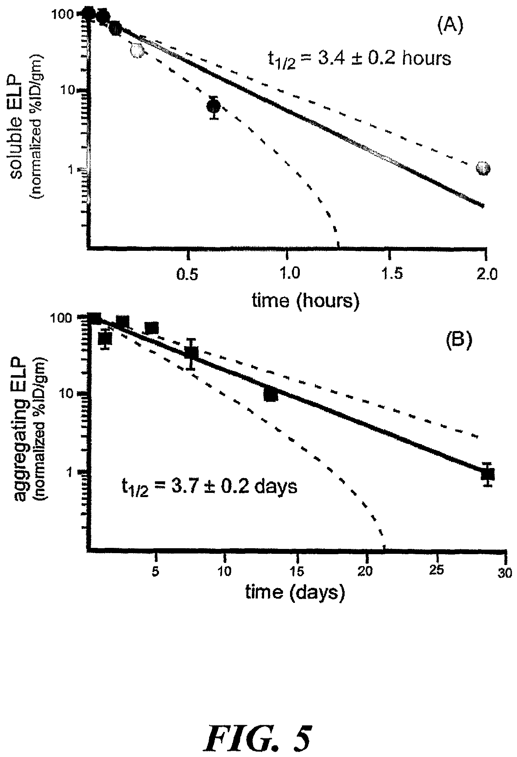

**Figure 5****.** Biodistribution of [¹⁴C]ELP in the injected joint compartment over time. The injected dose (ID/gm) is amount of ¹⁴C recovered from the injected knee compartment (per gram of recovered tissues and fluids) at time zero (10 min post injection); ID/gm at subsequent time points was normalized by this value to determine a %ID/gm for the knee joint compartment as a function of time. (A) Non-aggregating ELP (Tₜ > 50°C) and (B) aggregating ELP (Tₜ < 35°C). Data expressed as mean ± SE (n=5) were fit to a first-order exponential decay function (solid-line) and plotted on a semi-log axis. Confidence intervals (95%, dashed lines) are also shown. The time constant (τ) for each fit was used to calculate the joint half-life of the two ELP types [t_{1/2} = τ ln(2)).

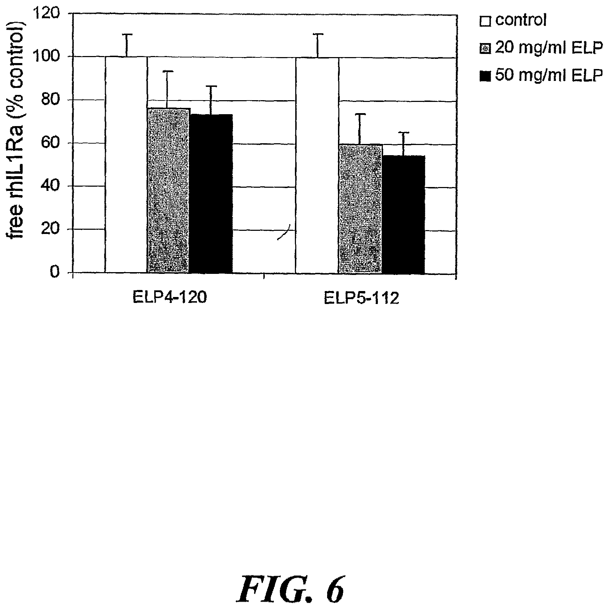

**Figure 6****.** Results for quantifying radiolabeled compound released to solution after mixing with ELP *in vitro.* [³H]rhIL1Ra at 2.5 micrograms/ml was mixed with different ELP formulations of different concentrations at 37°C (ELP4-120 ([(VPGVG)₁₀]₁₂, **SEQ ID NO:2**) and ELP5 ([(VPGVG)₆(VPGKG)]₁₆, **SEQ ID NO:3**)). ELP solutions were prepared at concentrations of 0 mg/ml (control), 20, and 50 mg/ml in a total reaction volume of 200 microliters. The ELP-ILIRa mixture was observed to complex into an aggregate. Aliquots of supernatant (5 microliters) were assayed over time for [³H] via scintillation counting as a measure of rhIL1Ra concentration in the supernatant. Measurements were normalized to control (0 mg/ml, hollow bar) to determine a %free rhIL1Ra in solution = (CPM of sample/CPM of control) x 100. Data are stable within 24h of entrapment; data shown here at 96 hours of equilibration (n=5, mean + SD). Results indicate that the ELPs are able to entrap between 25-55% of this compound upon mixing.

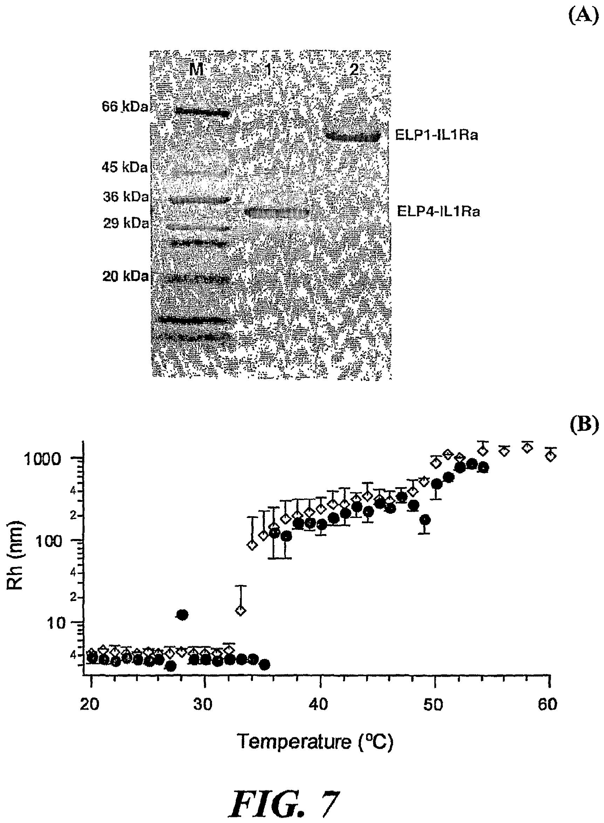

**Figure 7****.** (A) SDS-PAGE of two fusion proteins after two rounds of thermal purification. (B) Aggregation and particle size of the two fusion proteins with temperature were similar, showing the formation of large (> 100 nm) particles beginning at a temperature below 37°C.

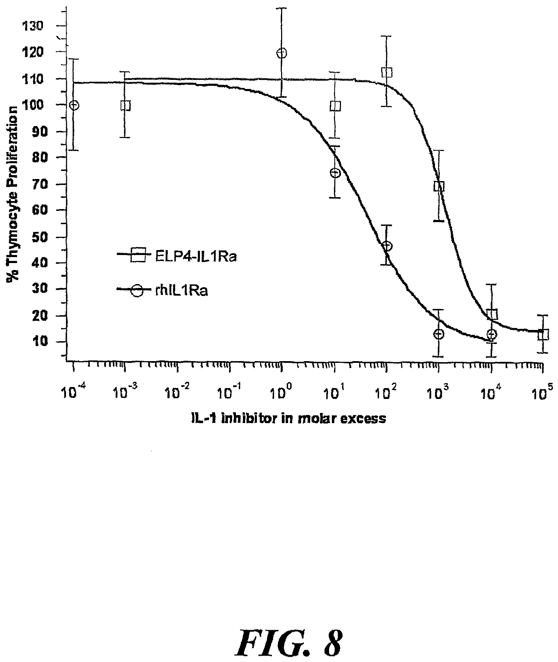

**Figure 8****.** Inhibition of Con A induced IL-1 dependent primary murine thymocytes proliferation by rhIL1Ra or ELP4-IL1Ra fusion protein. Cells cultured for 48 hours with 1 ng/ml murine IL-1β and increasing concentrations of fusion-protein or rhILIRa. Data presented as semi-log plots with the percentage of control proliferation vs. the amount of IL-1 inhibitor added in molar excess over the amount of IL-1 β present. Mean ± SD (n=6), data fit to a sigmoid function to determine the IC50 for each protein.

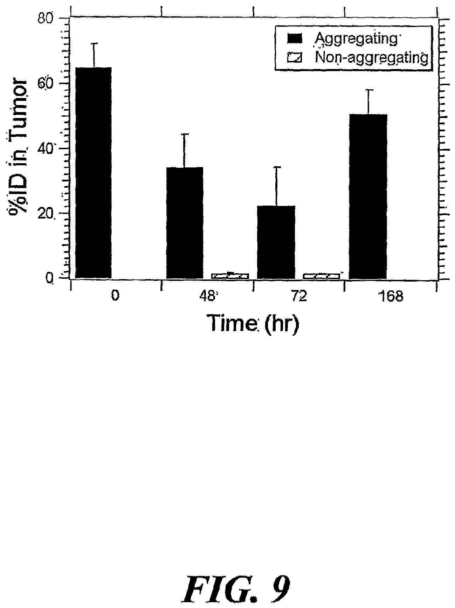

**Figure 9****.** Percent injected dose (%ID) in a tumor of aggregating (solid bar) and non-aggregating (hatched bar) ELPs. Both types of ELPs were infused (4 µL/min) into a human squamous cell carcinoma (FaDu) tumor on the leg of a nude mouse (Balb/c nu/nu). Data are mean ± SEM, n = 1 to 9.

### Detailed Description of the Preferred Embodiments

"Bioelastic polymer" as used herein refers to compounds that comprise repeating elastomeric units. The elastomeric units are typically pentapeptides, tetrapeptides, or nonapeptides. Examples are elastin-like polypeptides or "ELPs".

"Subjects" as used herein includes human subjects (including both male and female subjects and including juvenile, adolescent, adult, and geriatric subjects), as well as animal subjects, particularly other mammalian subjects such as primates, dogs, cats, and horses, for veterinary purposes.

"Arthritis" as used herein means any type of arthritis, including but not limited to rheumatoid arthritis, osteoarthritis, and ankylosing spondylitis.

"Aggregate" as used herein means a collection or agglomeration of a plurality of molecules such that a particle is formed.

"Dis-aggregation" as used herein means a separation of molecule or molecules from an aggregate, a progressive diminishing of aggregate size; or a progressive diminishing of particle size.

"Region of interest" as used herein may be any region of interest, including but not limited to joints and solid tumors ("solid tumor" including the resection cavities within solid tumors after surgical removal of the primary mass thereof and into which the compositions of the invention may be directly administered). In some embodiments of joint administration, the region of interest is an intervetebral disk space or a related spinal joint structure.

"Body temperature" as used herein includes both core body temperature and regional body temperature (*e.g.,* the temperature of an extremity when a region of interest such as a joint is located in an extremity).

"Joint" as used herein refers to a movable point where two bones meet, often a synovial joint or syndesmosis, and including but not limited to ball and socket joints, ellipsoidal joints, gliding joints, hinge joints, pivot joints and saddle joints. Such joints may be located in, for example, the shoulder, neck, spine, elbow, hip, wrist, hand, knee, ankle, or foot

."Solid tumor" as used herein may be any solid tumor or cancer, including but not limited to primary and secondary (or metastatic) solid tumors of lung cancer, liver cancer, prostate cancer, ovarian cancer, colon cancer, skin cancer (including melanoma), brain cancer, etc.

"Injection" or "injecting" as used herein may be carried out by any suitable means through a needle, syringe, shunt, cannula (*e.g.,* of 7-33 gauge) or any other suitable device that delivers the composition to be delivered directly into the region of interest (in contrast to and not including systemic delivery), as a single bolus or as an infusion over time.

"Antibody" or "antibodies" as used herein refers to all types of immunoglobulins, including IgG, IgM, IgA, IgD, and IgE. The term "immunoglobulin" includes the subtypes of these immunoglobulins, such as IgG₁, IgG₂, IgG₃, IgG₄, etc. Of these immunoglobulins, IgM and IgG are preferred, and IgG is particularly preferred. The antibodies may be of any species of origin, including (for example) mouse, rat, rabbit, horse, or human, or may be chimeric antibodies. The term "antibody" as used herein includes antibody fragments which retain the capability of binding to a target antigen, for example, Fab, F(ab')₂, and Fv fragments, and the corresponding fragments obtained from antibodies other than IgG. Such fragments are also produced by known techniques.

"Chemotherapeutic agent" (or "anticancer agent") as used herein includes but is not limited to methotrexate, daunomycin, mitomycin, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamosifen, paclitaxel, vincristin, vinblastine, camptothecin, actinomycin D, and cytarabine.

The disclosures of all United States patent refrences cited herein are to be incorporated by reference herein in their entirety.

***Compounds of interest.*** Any suitable compound, including but not limited to proteins and peptides, may be coupled to the polymer (*e.g.,* via chemical bond or production as a fusion protein) or mixed with the polymer as noted above. In general, the compounds of interest may be detectable groups or a therapeutic group.

For example, the compound of interest may be selected from the group of bone morphogenetic proteins, peptides, and growth factors, more particularly human calcitonin analogs, osteogenic growth peptides, and osteogenic growth peptide-human calcitonin analog hybrids. See, e.g., US Patent No. 6,593,394.

The compound of interest may be selected from the group of antiinfectives such as antibiotics and antiviral agents; chemotherapeutic agents (i.e. anticancer agents); anti-rejection agents; analgesics and analgesic combinations; anti-inflammatory agents; hormones such as steroids; growth factors, including bone morphogenic proteins (i.e. BMP's 1-7), bone morphogenic-like proteins (i.e. GDF-5, GDF-7 and GDF-8), epidermal growth factor (EGF), fibroblast growth factor (i.e. FGF 1-9), platelet derived growth factor (PDGF), insulin like growth factor (IGF-I and IGF-II), transforming growth factors (i.e. TGF-.beta. I-III), vascular endothelial growth factor (VEGF); and other naturally derived or genetically engineered proteins, polysaccharides, glycoproteins, or lipoproteins. See, e.g., D. Overaker, US Patent No. 6,575,986.

The compound of interest may be compounds or agents that actually promote or expedite healing, the effectors may also include compounds or agents that prevent infection (e.g., antimicrobial agents and antibiotics), compounds or agents that reduce inflammation (e.g., anti-inflammatory agents), compounds that prevent or minimize adhesion formation, such as oxidized regenerated cellulose (e.g., INTERCEED™ and SURGICEL™, available from Ethicon, Inc.), hyaluronic acid, and compounds or agents that suppress the immune system (e.g., immunosuppressants). Suitable compounds of interest include heterologous or autologous growth factors, proteins (including matrix proteins), peptides, antibodies, enzymes, platelets, glycoproteins, hormones, cytokines, glycosaminoglycans, nucleic acids, analgesics, viruses, virus particles, and cell types. It is understood that one or more effectors of the same or different functionality may be incorporated within the scaffold. Suitable compounds of interest include the multitude of heterologous or autologous growth factors known to promote healing and/or regeneration of injured or damaged tissue. Suitable compounds of interest include chemotactic agents; therapeutic agents (e.g., antibiotics, steroidal and non-steroidal analgesics and anti-inflammatories, anti-rejection agents such as immunosuppressants and anti-cancer drugs); various proteins (e.g., short term peptides, bone morphogenic proteins, glycoprotein and lipoprotein); cell attachment mediators; biologically active ligands; integrin binding sequence; ligands; various growth and/or differentiation agents and fragments thereof (e.g., epidermal growth factor (EGF), hepatocyte growth factor (HGF), IGF-I, IGF-II, TGF-.beta. I-III, growth and differentiation factors, vascular endothelial growth factors (VEGF), fibroblast growth factors (FGF), platelet derived growth factors (PDGF), insulin derived growth factor (IGF) and transforming growth factors, parathyroid hormone, parathyroid hormone related peptide, bFGF; TGFB superfamily factors; BMP-2; BMP-4; BMP-6; BMP-12; sonic hedgehog; GDF5; GDF6; GDF8; MP52, CDMP1); small molecules that affect the upregulation of specific growth factors; tenascin-C; hyaluronic acid; chondroitin sulfate; fibronectin; decorin; thromboelastin; thrombin-derived peptides; heparin-binding domains; heparin; heparan sulfate; DNA fragments and DNA plasmids. Suitable effectors likewise include the agonists and antagonists of the agents noted above. The growth factor can also include combinations of the growth factors listed above. In addition, the growth factor can be autologous growth factor that is supplied by platelets in the blood. In this case, the growth factor from platelets will be an undefined cocktail of various growth factors. *See, e.g.,* J. Hwang et al., US Patent Application Publication No. 2004/0267362.

In some embodiments, particularly for the treatment of arthritis, the compound of interest is an antiinflammatory agent, examples of which include but are not limited to TNF antibodies, IL-1 antibodies, soluble TNF receptors, soluble IL-1 receptors, TNF receptor antagonists, IL-1 receptor antagonists, COX-2 inhibitors, and non-steroidal antiinflammatory agents (including active fragments thereof for protein or peptide compounds such as antibodies and receptors). Numerous examples of such compounds are known. *See, e.g.,* US Patent No. 6,846,834; *see also* US Patent Nos. 6,624,184; 6,596,746; 6,498,165; and 6,420,373. One particular example is rhIL1Ra (including its isoforms)(commercially available as KINERET™ from Amgen), as described in US Patents Nos. 6,599,873 and 5,075,222.

***Bioelastic polymers.*** Bioelastic polymers are known and described in, for example, US Patent No. 5,520,672 to Urry et al. In general, bioelastic polymers are polypeptides comprising elastomeric units of bioelastic pentapeptides, tetrapeptides, and/or nonapeptides. Thus in some embodiments the elastomeric unit is a pentapeptide, in other embodiments the elastomeric unit is a tetrapeptide, and in still other embodiments the elastomeric unit is a nonapeptide. Bioelastic polymers that may be used to carry out the present invention are set forth in U.S. Pat. No. 4,474,851, which describes a number of tetrapeptide and pentapeptide repeating units that can be used to form a bioelastic polymer. Specific bioelastic polymers that can be used to carry out the present invention are also described in U.S. Pat. Nos. 4,132,746; 4,187,852; 4,500,700; 4,589,882; and 4,870,055. Still other examples of bioelastic polymers are set forth in US Patent No. 6,699,294 to Urry, US Patent No. 6,753,311 to Fertala and Ko, US Patent No. 6,063,061 to Wallace and US Patent No. 6,852,834 to Chilkoti. The bioelastic polymers may contain additional residues or units such as leader and/or trailer sequences as is known in the art.

In one nonlimiting example, the bioelastic polymers used to carry out the present invention are polypeptides that contain at least the general block structure, [(VPGXⁱG)ₙⁱ]ₘ (**SEQ ID NO:4**) for i=1 to J, Xⁱ is any amino acid other than proline (*e.g.,* Ala, Leu, Phe), nⁱ is the number of successive repeats of block i adjacent to and preceding block i+1. nⁱ is any number such as 1, 2, 3, or 4 up to 20 and nⁱ are independent of i. J is any number such as 1, 2, or 3 to 5 or more. m is the number of repeats of the block structure where m is any suitable number such as 2, 3 or 4 up to 60, 80 or 300 or more. The identity and frequency of the various amino acids at the fourth position can be changed. For example, the bioelastic polymers used to carry out the present invention may be polypeptides of the general formula: [(VPGAG)₅¹(VPGVG)²(VPGGG)₂³]ₘ, (**SEQ ID NO:5**) where X¹ = A, X² = V, X³ = G, n¹ is 5, n² is 1, n³ is 2, m is at least 1, 2, or 3 up to 100, 150 or 300 or more.

In another nonlimiting example, bioelastic polymers used to carry out the present invention may comprise repeating elastomeric units selected from the group consisting of bioelastic nonpeptides, pentapeptides and tetrapeptides, where the repeating units comprise amino acid residues selected from the group consisting of all amino acid and glycine residues. Preferred amino acid residues are selected from the group consisting of alanine, valine, leucine, isoleucine, proline, phenylalanine, tryptophan, and methionine. In many cases, the first amino acid residue of the repeating unit is a residue of valine, leucine, isoleucine or phenylalanine; the second amino acid residue is a residue of proline; the third amino acid residue is a residue of glycine; and the fourth amino acid residue is glycine or a very hydrophobic residue such as tryptophan, phenylalanine or tyrosine. Particular examples include the tetrapeptide Val-Pro-Gly-Gly (**SEQ ID NO:6**), the tetrapeptide GGVP (**SEQ ID NO:7**), the tetrapeptide GGFP (**SEQ ID NO:8**), the tetrapeptide GGAP (**SEQ ID NO:9**), the pentapeptide is Val-Pro-Gly-Val-Gly (**SEQ ID NO:10**), the pentapeptide GVGVP (**SEQ ID NO:11**), the pentapeptide GKGVP (**SEQ ID NO:12**), the pentapeptide GVGFP (**SEQ ID NO:13**), the pentapeptide GFGFP (**SEQ ID NO:14**), the pentapeptide GEGVP (**SEQ ID NO:15**), the pentapeptide GFGVP (**SEQ ID NO:16**), and the pentapeptide GVGIP (**SEQ ID NO:17**). *See, e.g.,* US Patent No. 6,699,294 to Urry.

***Administration.*** As noted above, a method of the invention generally comprises administering a composition to region of interest (*e.g.,* directly, such as by injection in or to the region of interest). The composition comprises the compound to be delivered and a polymer that undergoes an inverse temperature phase transition. The polymer has a transition temperature (Tₜ) less than the body temperature of the subject (*e.g.,* less than 37°C). The conjugate aggregates in the region of interest and then gradually disaggregates, providing a sustained release of the compound of interest at the selected region. The administering step may be carried out by any suitable means, such as injection, and particularly intra-articular injection (*e.g.,* into the synovial fluid of a synovial joint).

Patients afflicted with any disease or condition for which a depot drug is desired can be treated by the methods, with a particular example being patients afflicted with arthritis.

The compound of interest can be administered in any suitable amount depending upon the site of injection, age, weight, and condition of the subject, particular active agent, etc. In general the compound is administered in an amount of from 0.001, 0.01, 0.1, 1, 5 or 10 milligrams per administration or injection, up to about 100, 200 or 300 milligrams per administration or injection. In general the compound is administered at a concentration of 1 pg/ml to 500 mg/ml, with the injection volume dependent upon the region of interest. In general the ELP may also be co-administered at an equivalent concentration of 1 pg/ml to 500 mg/ml, or much higher concentrations in the example of mixing compound with elastomer. In some embodiments the compound of interest preferably has an *in vivo* half life in said region of one or two hours or more; in some embodiments administration of the compound of interest in combination with ELP in the said administration method is associated with an *in vivo* half life for the compound in said region of at least 2 or 3 days, one week, or four weeks or more, up to 2 or three months or more. Stated otherwise, it is contemplated that the administering step will be carried out on a regular and repeated basis (*e.g.,* for the treatment of a chronic condition such as arthritis), but that the frequency of administration will not be greater than, one, two, three or four times per month, while still retaining the desired treatment-effective amount.

The present invention is explained in greater detail in the following non-limiting Examples.

### EXAMPLES

Elastin like polypeptides (ELPs) are genetically engineered biopolymers, made up of the pentapeptide repeat unit as defined above that undergo aggregation, in response to an increase in temperature. ELPs are soluble at temperatures below their transition temperature (Tₜ), and become insoluble and form micron size aggregates at temperatures above their Tₜ. **We disclose herein that a protein drug attached to this drug carrier will aggregate at the time of injection and form a drug *'depot'* at the injection site, which will then slowly disaggregate into the joint space in a sustained manner** (*see, e.g.,* **Figure 1****).** In these examples we will evaluate the *in vitro* disaggregation of ELP at a temperature above its Tₜ, as well as the *in vivo* biodistribution and joint half-life of aggregating (Tₜ < 30°C) and non-aggregating (Tₜ > 50°C) ELPs after intra-articular injection. Furthermore, we will design fusion proteins of ELP and IL1Ra and evaluate their binding affinity to the IL-1 receptor and biological activity to inhibit IL-1 to demonstrate proof-of-concept for the ELP fusion protein drug carrier system.

### Example 1

### In Vitro ELP Disaggregation and Peptide Release

Elastin-like polypeptides (ELPs) or bioelastic polymers were evaluated for an ability to provide sustained release of "free" polymer from aggregate form as a proof-of-concept of the proposed delivery system for the treatment of localized diseases, such as osteoarthritis. The drug delivery system utilizes the unique ELP properties of rapid thermally-induced aggregation to form large particles, followed by sustained release of the ELP from the bulk aggregated large particles, termed a "free" form. To evaluate this, two ELP molecules were synthesized utilizing genetic engineering techniques and thermally purified: (1) ELP4-90 = [(VPGVG)₁₀]₉ (**SEQ ID NO:1**), Tₜ = 30.6°C, MW = 37 kDa; and (2) ELP4-120 = [(VPGVG)₁₀]₁₂ (**SEQ ID NO:2**), Tₜ = 28.6°C, MW = 49 kDa, in accordance to known techniques (*see* US Patent No. 6,699, 294 to Urry and US Patent No. 6,852,834 to Chilkoti). For the *in vitro* study, 1.5 ml of either ELP at a concentration of 30 mg/ml was placed in conical tubes and incubated at 37°C for 24h. At the end of this time, the supernatant was completely exchanged for PBS at 37°C and the quantity of ELP in the supernatant quantified as a function of starting ELP (**Figure 2****,** t = 0 hours) via absorbance at 280 nm. The sample was returned to 37°C and the amount of free ELP was quantified in 7 µl aliquots of supernatant over time. A time constant for equilibration was determined by numerically fitting data for free ELP concentration to a first-order exponential function.

For both ELP types, the polypeptide underwent an inverse temperature phase transition shortly after being placed at 37°C and slowly reached equilibrium with 15-20% of the starting ELP in a free form (**Figure 2**). The time constants for equilibration were on the order of 1-2 days after the supernatant was completely exchanged. The equilibrium constant describing steady-state values for free to aggregate ELP concentrations were between 0.15-0.20. These results illustrate that an aggregated ELP form will slowly release free ELP over time. In a diarthrodial joint where the free form will be constantly cleared through the synovial tissue, promoting continuous "disaggregation" of the ELP aggregate.

### Example 2

### Biodistribution of ELP After Intra-articular Injection

The biodistribution and joint half-life of an aggregating ELP4-120 ([(VPGVG)₁₀]₁₂, **SEQ ID NO:2,** Tₜ < 32°C, MW 47 kDa) and non-aggregating, ELP2 ([(VPGVG)₁(VPGAG)₈(VPGGG)₇]₁₀, **SEQ ID NO:18,** MW 61 kDa, Tₜ > 50°C). ELPs following intra-articular injection were evaluated in a rat animal model. The ELPs were labeled with [¹⁴C] to yield a specific activity between 32-37 mCi/mmole. The labeled ELP was dialyzed, sterile filtered (0.22 µm filter) and a volume of 30 µl at ∼650 µM of either peptide was injected into the joint cavity of the right knee of Wistar rats. After injection, the animals were housed in a cage for up to 4 weeks. At each time point five rats were sacrificed, their right and left knee (synovial fluid, meniscus, cartilage, synovium), blood, heart, lung, liver, spleen, kidney, and bladder were collected and digested. The radioactivity of each tissue was determined using liquid beta-scintillation counting. Total counts per tissue weight were measured for each animal and for each tissue or body fluid (n=5 animals per group and per time point) and plotted for each tissue as a function of time. Values for joint tissues and joint fluids were summed into a total "joint" value (synovial fluid, meniscus, joint cartilages, synovium).

The total radioactivity recovered from all tissues and fluids in the joint cavity at 10 min after injection was adjusted for injected [¹⁴C]-ELP that was non-specifically distributed, with the starting dose found to be between 70-75% of administered dose. Radioactivity per tissue weight was then normalized by this initial injected knee joint cavity value (10 min post-injection), in order to obtain the percent of injected dose per gram (%ID/gm) of tissue relative to the delivered amount for all recovered tissues and fluids.

At all times, no preferential accumulation of either peptide in the peripheral organs was observed (**Figures 3C** or **4C**). Except in the injected knee and blood, the total amount of each peptide was less than 1% ID/gm in all other organs, (Figures **3B** or **4B**). The profile of non-aggregating ELP in the injected knee is shown in **Figure 3** (plotted against time in hours) and that of aggregating ELP in the injected knee is plotted in **Figure 4** (plotted against time in days). The non-aggregating ELP was below 10%ID/gm in the joint space within the first 24 hours, while the aggregating ELP took nearly two weeks to reach the same level.

A radioactive half-life (t_{1/2}) was determined for the injected knee joint compartment by summing the total radioactivity count for the joint as described (**Figure 5**). **Figure 5A** shows injected knee joint compartment values for non-aggregating ELP (Tₜ > 50°C) and 5B showing values for aggregating ELP (Tₜ < 35°C). Data expressed as mean ± SE (n=5) were fit to a first-order exponential decay function (solid-line) and plotted on a semi-log axis. Confidence intervals (95%, dashed lines) are also shown. The time constant (τ) for each fit was used to calculate the joint half-life of the two ELP types [t_{1/2} = τ ln(2)). The half-life of non-aggregating and aggregating ELP in the joint was found to be 3.4 ± 0.2 hours and 3.7 + 0.2 days, respectively. This suggests that the aggregation property of ELP significantly prolongs the half-life of the peptide in articular joints, by over 24 fold (p<0.05, ANOVA), confirming the proposed sustained release mechanism. These findings are significant because they demonstrate that the thermal responsive properties of ELP can be utilized to deliver protein drugs to arthritis-affected joints.

### Example 3

### Demonstration of Entrapment

**Figure 6** demonstrates entrapment by the methods and compositions of the present invention. **Figure 6** shows results for quantifying radiolabeled compound released to solution after mixing with ELP *in vitro.* [³H]rhIL1Ra at 2.5 micrograms/ml was mixed with different ELP formulations of different concentrations at 37°C (ELP4-120 [(VPGVG)₁₀]₁₂, **SEQ ID NO:2** and ELP5 [(VPGVG)₆(VPGKG)]₁₆, **SEQ ID NO:19**). ELP solutions were prepared at concentrations of 0 mg/ml (control), 20, and 50 mg/ml in a total reaction volume of 200 microliters. The ELP-IL1Ra mixture was observed to complex into an aggregate. Aliquots of supernatant (5 microliters) were assayed over time for [³H] via scintillation counting as a measure of rhILIRa concentration in the supernatant. Measurements were normalized to control (0 mg/ml) to determine a %free rhILIRa in solution = (CPM of sample/CPM of control) x 100. Data are stable within 24h of entrapment; data shown here at 96 hours of equilibration. Results indicate that the ELPs are able to entrap between 25-55% of this compound upon mixing.

### Example 4

### ELP-IL1Ra fusion protein design, synthesis and characterization

In proof-of-concept tests of this drug delivery system, two aggregating fusion proteins of ELP and IL1Ra were genetically synthesized as follows. The gene for human IL1Ra was PCR-cloned from LPS activated U937 cells as previously described by Carter et al. (1990) Nature 344: 633-8. Two ELP formulations were synthesized as described by US Patent No. 6,852,834 to Chilkoti: ELP1 [(VPGVG)₅(VPGGG)₃(VPGAG)₂]₉, (**SEQ ID NO:20**), MW 36 kDa, and ELP4 [(VPGVG)₁₀]₃, (**SEQ ID NO:21**), MW 14.5 kDa. Each ELP gene was then sub-cloned into the ILIRa containing vector (pET-25b+) using traditional molecular biology techniques to produce two ELP-IL1Ra fusion proteins (see **Tables 1-2**) with molecular weights of 30.2 and 53 kDa. The fusion proteins were then expressed in *E. coli* and thermally purified in accordance with known technique US Patent No. 6,699, 294 to Urry and US Patent No. 6,852,834 to Chilkoti. The size and purity of the fusion proteins was examined by SDS-PAGE and shown in **Figure 7A****.** The thermal purification method used here yielded > 50 mg fusion protein /liter of bacterial growth with > 95% purity.

The temperature driven aggregation of each fusion protein was evaluated by dynamic light scattering, where the scattering of laser light by the fusion protein was monitored while a dilute solution (25 µM) of the fusion protein was heated at 1°C interval over the range of 20 - 60°C. From the scatter data the hydrodynamic radius (Rₕ) was determined by the Stokes-Einstein relationship for spherical particles. The aggregation temperature was defined as the temperature above which large (>100 nm) particles start to form and the fusion protein aggregates. **Figure 7B** shows that the designed fusion proteins undergo temperature driven phase transition and form large aggregates. The aggregates start to form at a temperature below 37°C. This thermal responsive behavior is an important part of the drug delivery system; it implies that the designed fusion proteins of ELP and ILIRa will aggregate at the time and site of injection, which leads to the increased half-life of the protein drug, IL1Ra.

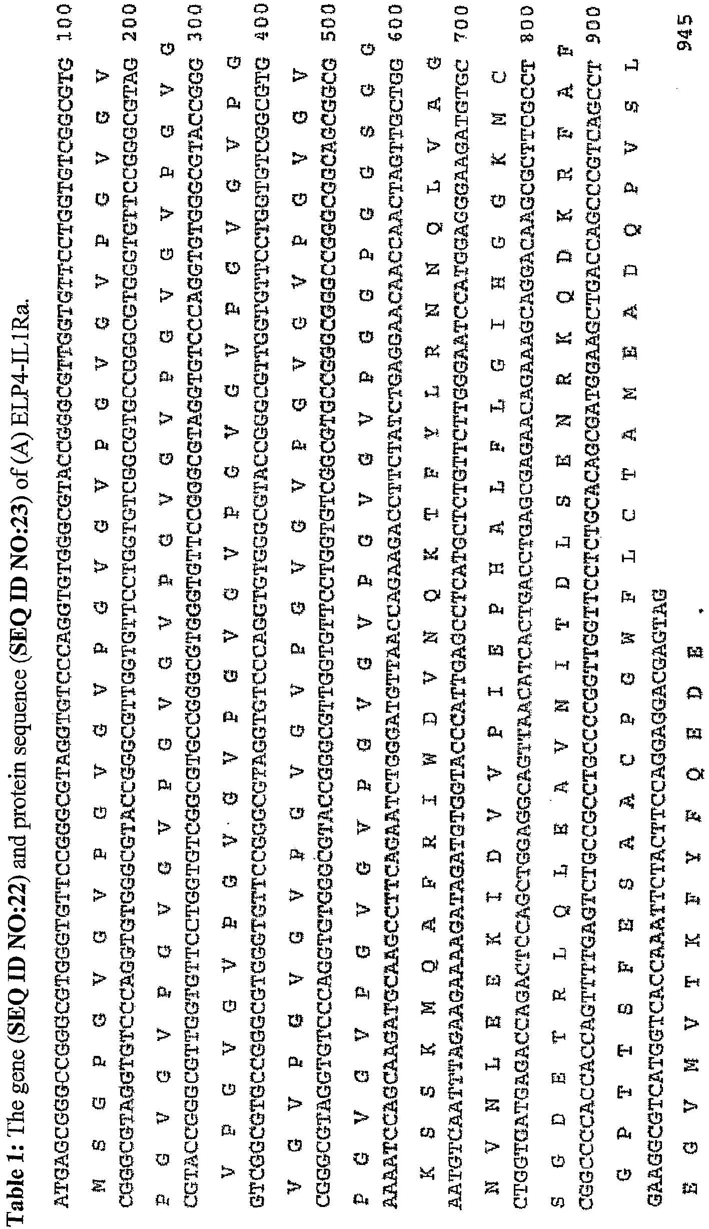

(The ELP4 gene spans the first 462 nucleotides followed by 33 base long spacer. The IL1Ra gene spans 492 to 942 bases)

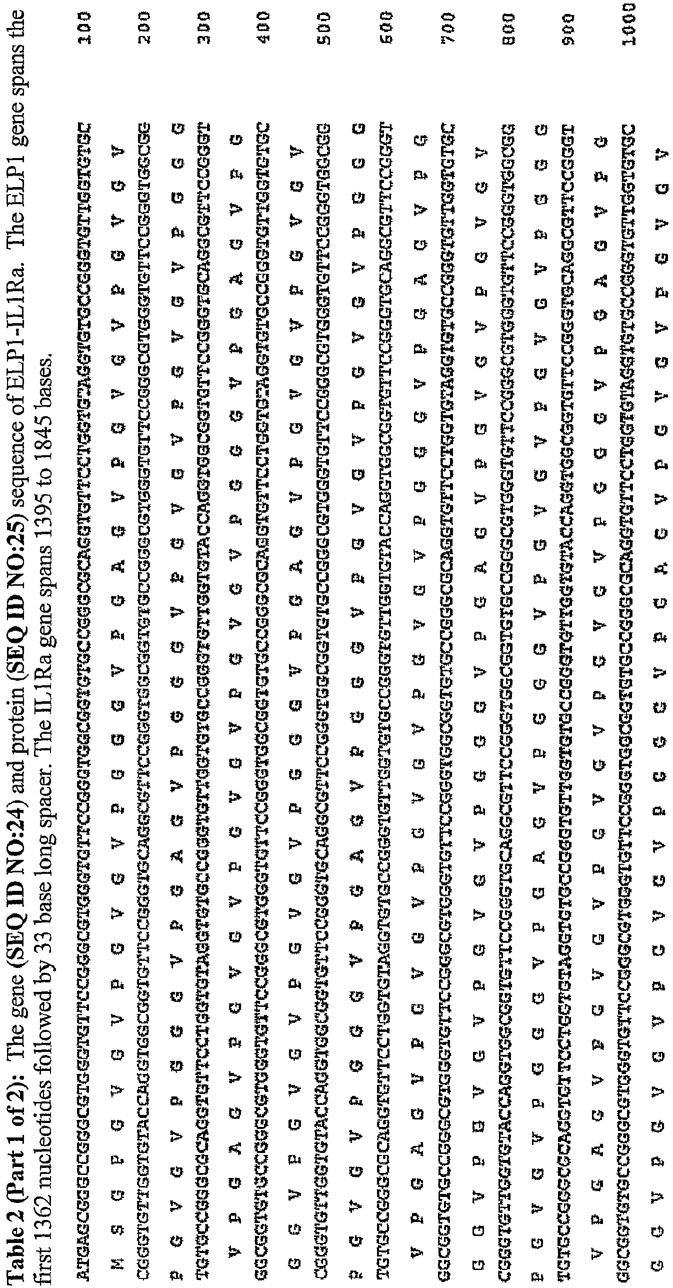

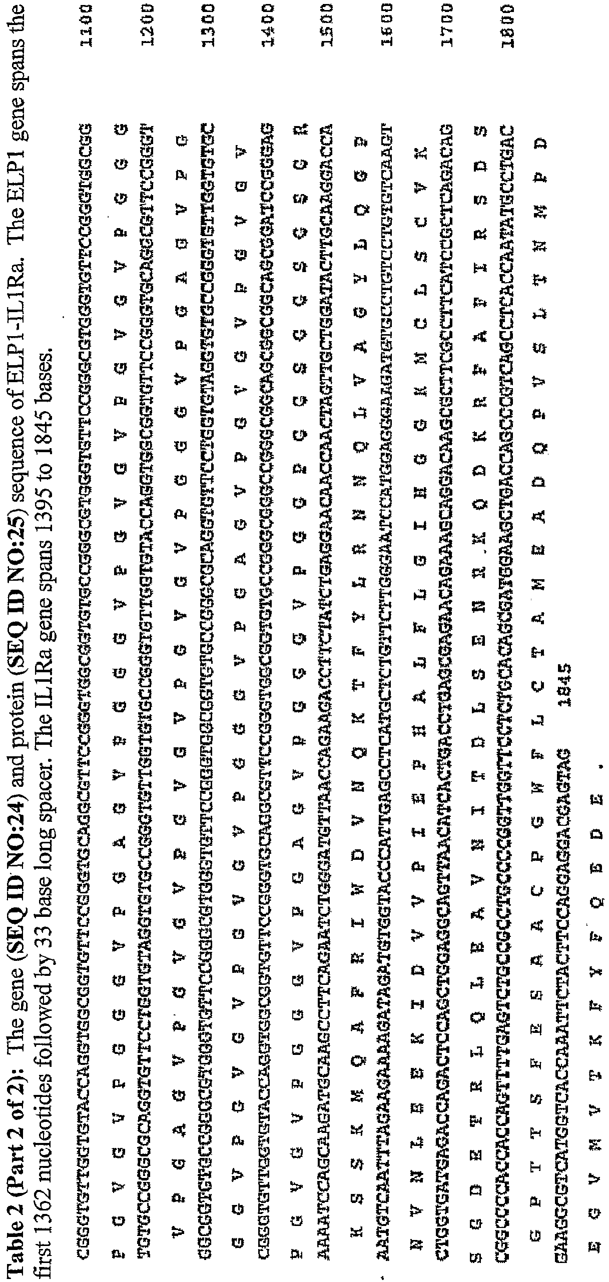

**(SEQ ID NO: 2)** The ELP1 gene spans the first 1362 nucleotides followed by 33 base long spacer. The IL1Ra gene spans 1395 to 1845 bases.

### Example 5

### Biological Activity of ELP-IL1Ra Fusion Protein

The bioactivity of a represententative fusion protein (ELP4-IL1Ra) was examined for its ability to inhibit IL-1β augmented proliferation of Con A-stimulated murine C57 primary thymocytes as previously described. Briefly, primary thymocytes were mechanically isolated from freshly sacrificed 5 to 7 weeks old C57 mice and suspended in RPMI 1640 supplemented with 5 % FCS, 1 µg/ml ConA (Sigma) and 1 ng/ml rmIL-1β (Pierce) at a density of ∼2 x 10⁶ cells/ 200 µl. The cells were then incubated in the presence of serial dilutions of either commercial rhIL1Ra (R&D Systems) or ELP4-IL1Ra fusion protein (as described above) for 48 hours. The proliferation of thymocytes was assessed by the uptake of [³H]-thymidine (0.5 µCi/well). Data were expressed as mean CPM ± SD of [³H]-thymidine uptake of six replicate cultures. The highest concentration producing 50% inhibition (IC50) was determined by fitting the data to a sigmoid function. The ELP4-IL1Ra fusion protein showed significant bioactivity with an IC50 of 61.4 ± 19.8 nM to inhibit 52 pM rmIL-1β, see **Figure 8****.**

### Example 6

### Tumor administration

Examples of administration of compositions of the invention are given in **Figure 9****.** Percent injected dose (%ID) in a tumor of aggregating (solid bar) and non-aggregating (hatched bar) ELPs is shown. Both types of ELPs were infused (4 µL/min) into a human squamous cell carcinoma (FaDu) tumor on the leg of a nude mouse (Balb/c nu/nu). Data are mean ± SEM, n = 1 to 9. Note the far greater dose for aggregating, as compared to non-aggregating, ELPs.

The foregoing is illustrative of the present invention, and is not to be construed as limiting thereof. The invention is defined by the following claims, with equivalents of the claims to be included therein.

The following section of the description consists of numbered paragraphs simply providing statements of the invention already described herein. The numbered paragraphs in this section are not claims. The claims are set forth below in the later section headed "claims".
1. A method for delivering a drug depot of a compound of interest to a selected region in a subject, said method comprising:
   administering a composition directly to said region of interest, said composition comprising said compound to be delivered and a polymer that undergoes an inverse temperature phase transition;
   so that a sustained release of said compound of interest at said selected region is provided;
   wherein said polymer has a transition temperature (Tt) less than the body temperature of said subject.
2. The method of 1, wherein said composition aggregates in the region of interest and then gradually disaggregates, thereby providing said sustained release of said compound of interest at said selected region.
3. The method of 1, wherein said administering step is carried out by injection,
4. The method of 1, wherein said administering step is carried out by injection through a needle or cannula, wherein said needle or cannula is of 7 to 33 gauge.
5. The method of 1, wherein said region of interest is a joint.
6. The method of 1, wherein said region of interest is a joint, said compound of interest is an antiinflammatory compound, said patient is afflicted with osteoarthritis, and said composition is administered in an amount effective to treat said osteoarthritis.
7. The method of 1, wherein said administering step is carried out by intra-articular injection.
8. The method of 1, wherein said patient is afflicted with diarthrodial joint disorder and/or intervertebral disc pathology.
9. The method of 1, wherein said region of interest is an intervertebral disc space.
10. The method of 1, wherein said region of interest is a solid tumor.
11. The method of 1, wherein said region of interest is a solid tumor with which said subject is afflicted, and said composition is administered in an amount effective to treat said solid tumor.
12. The method of 1, wherein said administering step is repeated on a plurality of occasions at a frequency not greater than three times a month.
13. The method of 1, wherein said composition comprises said compound to be delivered conjugated to said polymer that undergoes an inverse temperature phase transition.
14. The method of 1, wherein said polymer is a bioelastic polymer, said bioelastic polymer comprising elastomeric units selected from the group consisting of bioelastic pentapeptides, tetrapeptides, and nonapeptides.
15. The method of 1, wherein said compound of interest is a protein or peptide.
16. The method of 1, wherein said compound of interest is an antiinflammatory compound selected from the group consisting of TNF blocking antibodies, IL-1 antibodies, soluble TNF receptors, soluble IL-1 receptors, TNF receptor antagonists, and IL-1 receptor antagonists.
17. The method of 1, wherein said compound of interest is recombinant human IL-1 receptor antagonist.
18. The method of 1, wherein said therapeutic compound is conjugated to said polymer.
19. The method of 1, wherein said therapeutic compound is mixed with said polymer.
20. A pharmaceutically acceptable composition comprising a therapeutic compound in combination with a polymer that undergoes an inverse temperature phase transition.
21. The composition of 20, wherein said therapeutic compound is selected from the group consisting of antiinflammatory agents and chemotherapeutic agents.
22. The composition of 20, wherein said therapeutic compound is an antiinflammatory agent.
23. The composition of 20, wherein said therapeutic compound is an antiinflammatory agent selected from the group consisting of TNF antibodies, IL-1 antibodies, soluble TNF receptors, soluble IL-1 receptors, TNF receptor antagonists, and IL-1 receptor antagonists.
24. The composition of 20, wherein said therapeutic compound is recombinant human IL-1 receptor antagonist
25. The composition of 20, wherein said therapeutic compound is a chemotherapeutic agent.
26. The composition of 20, wherein said therapeutic compound is conjugated to said polymer.
27. The composition of 20, wherein said therapeutic compound is mixed with said polymer.
28. An injection device containing a composition of 20 in sterile injectable form.
29. The injection device of 28, wherein said injection device is a syringe.

## Claims

1. A pharmaceutically acceptable composition comprising therapeutic agent comprising a chemotherapeutic agent coupled to an elastin-like polypeptide (ELP) comprising [(VPGVG)5(VPGGG)3(VPGAG)2]9 (SEQ. ID. NO:20) for use in treating cancer in a subject in need thereof.

2. The pharmaceutical composition for use of claim 1, wherein said chemotherapeutic agent is selected from the group consisting of: methotrexate, daunomycin, mitomycin, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamosifen, paclitaxel, vincristin, vinblastine, camptothecin, actinomycin D, and cytarabine.

3. The pharmaceutical composition for use of claim 1 or claim 2, wherein the therapeutic agent is administered to the subject in a treatment-effective amount.

4. The pharmaceutical composition for use of claim 1, wherein the cancer is metastatic.

5. The pharmaceutical composition for use of claim 4, wherein the cancer is a solid cancer.

6. The pharmaceutical composition for use of claim 4 or 5, wherein the solid cancer is selected from the group consisting of lung cancer, colon cancer, brain cancer, liver cancer, prostate cancer, ovarian cancer, skin cancer, and melanoma.

7. The pharmaceutical composition for use of any one of claims 1 to 6, wherein the pharmaceutical composition is administered to the subject via injection.

8. The pharmaceutical composition for use of claim 7, wherein the pharmaceutical composition is injected systemically.

9. The pharmaceutical composition for use of claim 7, wherein the pharmaceutical composition is injected locally.

10. The pharmaceutical composition for use of any one of claims 1 to 9, wherein the subject is human.
